# EUROPEAN PATENT APPLICATION

(11) **EP 4 691 459 A1**
(43) Date of publication of application: **11.02.2026**
(21) Application number: 24785257.7
(22) Date of filing: 04.04.2024
(51) Int. Cl.: A61K 9/24, A61K 31/41, A61K 31/225, A61K 31/7034, A61K 45/06, A61P 9/04

(54) **COMBINATION FORMULATION COMPRISING SACUBITRIL-VALSARTAN AND SGLT-2 INHIBITOR HAVING IMPROVED STABILITY AND DISSOLUTION RATE**

(30) Priority: 05.04.2023 KR 20230044631; 22.03.2024 TW 113110861
(71) Applicant: Hanmi Pharm. Co., Ltd., Hwaseong-si, Gyeonggi-do 18536 (KR)
(72) Inventor: CHOI, Min Jong, Hwaseong-si Gyeonggi-do 18536 (KR); SIM, Sung Bo, Hwaseong-si Gyeonggi-do 18536 (KR); KWON, Taek Kwan, Hwaseong-si Gyeonggi-do 18536 (KR); LEE, Dong Wook, Hwaseong-si Gyeonggi-do 18536 (KR)
(74) Representative: Schnappauf, Georg
(86) International application number: PCT/KR2024/004409
(87) International publication number: WO 2024/210557

(57) **Abstract**

The present invention relates to a pharmaceutical combination formulation which has a synergistic effect in the treatment of heart failure by containing both sacubitril-valsartan and an SGLT-2 inhibitor as active ingredients, and at the same time, ensures bioequivalence between the drugs because the effect of one drug on the dissolution of the other drug is minimized. In addition, the generation of impurities in the pharmaceutical combination formulation is inhibited, and thus the stability of the active ingredients is also excellent.

## Description

### TECHNICAL FIELD

The present invention relates to a combination formulation containing sacubitril-valsartan and an SGLT-2 inhibitor as active ingredients and having excellent stability and dissolution rate.

### BACKGROUND ART

Heart failure refers to a condition in which the filling function (diastolic function) of the heart to receive blood or the pump function (systolic function) of the heart to squeeze blood is reduced due to a structural or functional abnormality of the heart, and as a result, the heart does not properly supply the necessary blood to the body's tissues. When heart failure occurs, a decrease in myocardial function, remodeling of the left ventricle, hemodynamic changes, activation of the neurohormonal system, expression of cytokines, dysfunction of vascular endothelial cells, etc. may occur regardless of the cause, leading to various types of complications such as arrhythmia, atrial fibrillation, stroke, acute pulmonary edema, kidney function damage, and sudden death.

While the number of patients with heart failure, especially heart failure with preserved ejection fraction (HFpEF), is increasing worldwide, there is no approved therapeutic agent, and thus expectations are high for the development of related drugs. Recently, Entresto^{™} (ingredient name: sacubitril-valsartan combination formulation), a newly introduced heart failure therapeutic agent, has attracted attention as the first therapeutic agent that surpasses existing ACE inhibitors.

The active ingredient of Entresto^{™} is sacubitril-valsartan sodium hemipentahydrate, the content of which is disclosed in International Patent Publication No. WO 2007/056546 A1 (Patent Document 1) (the entire content of the patent document is hereby incorporated as prior art in the present specification). It has been reported that Entresto^{™} is effective in reducing death from cardiovascular disease and risk factors due to heart failure, but has poor stability and water solubility, and the efficiency of preparation thereof is very poor.

Meanwhile, recent large-scale clinical trials conducted in Korea and foreign countries have indicated that SGLT-2 inhibitors, previously released as anti-diabetic drugs, are effective in treating heart failure regardless of the presence or absence of diabetes. In the revised 2022 heart failure treatment guidelines recently published by the Korean Society of Heart Failure, sodium-glucose cotransporter 2 (SGLT2) inhibitors such as Farxiga^{™} and Jardiance^{™}, in addition to Entresto^{™}, were recommended as first-line standard treatment (Class I, evidence grade A).

Accordingly, the present inventors have sought to develop a combination formulation containing both sacubitril-valsartan and an SGLT2 inhibitor so that the effect thereof on the treatment of heart failure and ischemic heart disease can be increased, and as a result, have developed a combination form of the present invention, which enables immediate release of the two ingredients in consideration of the sites of action of these drugs while having excellent stability, thereby completing the present invention.

(Patent Document 1) International Patent Publication No. WO2007/056546

(Patent Document 2) U.S. Patent No. 6515117

### DISCLOSURE

### Technical Problem

An object of the present invention is to provide a combination formulation for oral administration that has a synergistic effect in the treatment of heart failure by containing both sacubitril-valsartan and an SGLT-2 inhibitor as active ingredients, and at the same time, can show dissolution profiles equivalent to those when these drugs are administered individually alone, and also has excellent stability.

Another object of the present invention is to provide a method for efficiently preparing the combination formulation for oral administration provided by the present invention.

However, objects to be achieved by the present invention are not limited to the objects mentioned above, and other objects not mentioned above can be clearly understood by those skilled in the art from the following description.

### Technical Solution

In accordance with one embodiment of the present invention, the present invention is directed to a pharmaceutical combination formulation comprising: (1) a first release portion comprising sacubitril-valsartan or pharmaceutically acceptable salts thereof as an active ingredient; and (2) a second release portion comprising a sodium-glucose cotransporter-2 (SGLT-2) inhibitor or a pharmaceutically acceptable salt thereof as an active ingredient.

In the present invention, the first release portion and the second release portion may be present in a state in which they are physically separated from each other.

In the present invention, the pharmaceutical combination formulation may be a multilayer tablet or a drug-coated tablet.

In the present invention, the SGLT-2 inhibitor may be selected from the group consisting of dapagliflozin, empagliflozin, canagliflozin, ertugliflozin, sotagliflozin, ipragliflozin, tofogliflozin, luseogliflozin, bexagliflozin, and remogliflozin.

In the present invention, the first or second release portion may comprise at least one pharmaceutically acceptable additive selected from the group consisting of an excipient, a disintegrant, and a lubricant.

In the present invention, the excipient may be selected from the group consisting of microcrystalline cellulose, silicified microcrystalline cellulose, magnesium aluminometasilicate, magnesium aluminosilicate, aluminum silicate, sodium silicate, potassium silicate, magnesium silicate, calcium silicate, lactose, lactose hydrate, anhydrous lactose, calcium hydrogen phosphate, hydroxypropyl cellulose, hydroxypropyl methylcellulose, dextrin, mannitol, sorbitol, starch, calcium phosphate hydrate, calcium carbonate, sugars, and mixtures thereof

In the present invention, the disintegrant may be selected from the group consisting of crospovidone, methylcellulose, cross-linked carboxymethylcellulose sodium (cross-linked CMC Na, C.CMC Na, or croscarmellose sodium), carboxymethylcellulose calcium, sodium starch glycolate, hydroxypropylcellulose, low-substituted hydroxypropylcellulose (L-HPC), hydroxypropyl methylcellulose, starch, pregelatinized starch, corn starch, potato starch, alginic acid or its sodium salt, and combinations thereof.

In the present invention, the lubricant may be selected from the group consisting of calcium stearate, colloidal silicon dioxide (fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and combinations thereof.

In the present invention, the second release portion may comprise a coating agent.

In the present invention, the coating agent may be at least one selected from the group consisting of hydroxypropyl methylcellulose (HPMC), polyvinyl alcohol (PVA), macrogol polyvinyl alcohol grafted copolymers, polymers of acrylic acid and its salts, polymethacrylate, poly(butylmethacrylate, 2-dimethylaminoethyl methacrylate, methyl methacrylate) copolymers, carboxymethylcellulose, ethylcellulose, methylcellulose, hydroxyethylcellulose, ethyl hydroxyethyl cellulose, hydroxypropylcellulose (HPC), L-HPC (low-substituted HPC), polyvinylpyrrolidone (PVP), vinylpyrrolidone-vinylacetate copolymers, gelatin, guar gum, partially hydrolyzed starch, alginate, xanthan, and mixtures thereof.

In the present invention, the first release portion may comprise, based on the total weight of the first release portion, 10 to 80 wt% of the active ingredient, 1 to 50 wt% of an excipient, 5 to 50 wt% of a disintegrant, and 0.1 to 20 wt% of a lubricant.

In the present invention, the second release portion may comprise, based on the total weight of the second release portion, 0.5 to 50 wt% of the active ingredient, 20 to 95 wt% of an excipient, 0.1 to 20 wt% of a disintegrant, and 0.1 to 20 wt% of a lubricant.

In the present invention, the second release portion may comprise, based on the total weight of the second release portion, 10 to 70 wt% of the active ingredient and 30 to 90 wt% of a coating agent.

In the present invention, the first release portion may comprise, based on the total weight of the first release portion, 10 to 80 wt% of the active ingredient, 5 to 50 wt% of at least one of microcrystalline cellulose and mannitol, 5 to 30 wt% of low-substituted hydroxypropyl cellulose, 5 to 30 wt% of at least one of sodium starch glycolate and croscarmellose sodium, 0.1 to 10 wt% of colloidal silicon dioxide, 0.1 to 10 wt% of talc, and 0.1 to 10 wt% of magnesium stearate.

In the present invention, the second release portion may comprise, based on the total weight of the second release portion, 0.5 to 50 wt% of the active ingredient, 10 to 50 wt% of at least one of microcrystalline cellulose and lactose hydrate, 10 to 50 wt% of mannitol, 0.1 to 20 wt% of hydroxypropyl cellulose, 0.5 to 20 wt% of croscarmellose sodium, and 0.5 to 20 wt% of sodium stearyl fumarate.

In the present invention, the pharmaceutical combination formulation may be a bilayer tablet comprising: a first layer comprising a first release portion comprising sacubitril-valsartan or pharmaceutically acceptable salts thereof as an active ingredient; and a second layer comprising a second release portion comprising an SGLT-2 inhibitor or a pharmaceutically acceptable salt thereof as an active ingredient.

In the present invention, the pharmaceutical combination formulation may be a drug-coated tablet comprising: a core comprising a first release portion comprising sacubitril-valsartan or pharmaceutically acceptable salts thereof as an active ingredient; and a coating layer located on at least a portion of the surface of the core and comprising a second release portion comprising an SGLT-2 inhibitor or a pharmaceutically acceptable salt thereof as an active ingredient.

In the present invention, the first release portion may comprise granules comprising sacubitril-valsartan or pharmaceutically acceptable salts thereof as an active ingredient, an excipient, a disintegrant, and a lubricant, and a post-mixture portion comprising a disintegrant and a lubricant, and
the second release portion may comprise granules comprising an SGLT-2 inhibitor or a pharmaceutically acceptable salt thereof as an active ingredient, an excipient, a disintegrant, and a lubricant, and a post-mixture portion comprising a disintegrant and a lubricant.

In accordance with another embodiment of the present invention, the present invention is directed to a method for preparing a pharmaceutical combination formulation, the method comprising steps of: (a) preparing a first release portion comprising sacubitril-valsartan or pharmaceutically acceptable salts thereof as an active ingredient; (b) preparing a second release portion comprising an SGLT-2 inhibitor or a pharmaceutically acceptable salt thereof as an active ingredient; and (c) formulating the first release portion and the second release portion into a single solid formulation.

In the present invention, step (a) may comprise steps of: preparing granules comprising sacubitril-valsartan or pharmaceutically acceptable salts thereof and a pharmaceutically acceptable additive; and mixing the granules with a post-mixture portion comprising a disintegrant and a lubricant, step (b) may comprise steps of: preparing granules comprising the SGLT-2 inhibitor or pharmaceutically acceptable salt thereof and a pharmaceutically acceptable additive; and mixing the granules with a post-mixture portion comprising a disintegrant and a lubricant, and step (c) may comprise compressing the mixture of step (a) and the mixture of step (b) into a bilayer tablet.

In the present invention, step (a) may comprise steps of: preparing granules comprising sacubitril-valsartan or pharmaceutically acceptable salts thereof and a pharmaceutically acceptable additive; mixing the granules with a post-mixture portion comprising a disintegrant and a lubricant; and compressing the mixture into an uncoated tablet, step (b) may comprise a step of preparing a coating solution by mixing the SGLT-2 inhibitor or pharmaceutically acceptable salt thereof with a coating agent, and step (c) may comprise a step of preparing a drug-coated tablet by coating the surface of the uncoated tablet of step (a) with the coating solution of step (b).

### Advantageous Effects

The oral combination formulation provided by the present invention may provide a pharmaceutical combination formulation which has a synergistic effect in the treatment of heart failure by comprising both sacubitril-valsartan and an SGLT-2 inhibitor as active ingredients, and at the same time, ensures bioequivalence because the effect of one drug on the dissolution of the other drug is minimized. In addition, the oral combination formulation of the present invention suppresses the generation of impurities and thus has excellent stability of the active ingredients.

### BRIEF DESCRIPTION OF DRAWINGS

FIG. 1 shows the results of an experiment comparing the dissolution rate of dapagliflozin in pH 1.2 buffer between combination formulations of Examples 1 to 3 and Comparative Examples 1 to 5 and a reference drug, in Experimental Example 1.
FIG. 2 shows the results of an experiment comparing the dissolution rate of sacubitril in pH 1.2 buffer between combination formulations of Examples 1 to 3 and Comparative Examples 1 to 5 and a reference drug, in Experimental Example 1.
FIG. 3 shows the results of an experiment comparing the dissolution rate of valsartan in pH 1.2 buffer between combination formulations of Examples 1 to 3 and Comparative Examples 1 to 5 and a reference drug, in Experimental Example 1.
FIG. 4 shows the results of an experiment comparing the dissolution rate of dapagliflozin in pH 6.8 buffer for combination formulations of Examples 1 to 3 and Comparative Examples 1 to 5 and a reference drug, in Experimental Example 1.
FIG. 5 shows the results of an experiment comparing the dissolution rate of sacubitril in pH 6.8 buffer between combination formulations of Examples 1 to 3 and Comparative Examples 1 to 5 and a reference drug, in Experimental Example 1.
FIG. 6 shows the results of an experiment comparing the dissolution rate of valsartan in pH 6.8 buffer between combination formulations of Examples 1 to 3 and Comparative Examples 1 to 5 and a reference drug, in Experimental Example 1.
FIG. 7 shows the results of an experiment comparing the amount of dapagliflozin impurity (at RRT 0.38) generated under initial, accelerated or stress conditions between combination formulations of Examples 1 to 3 and Comparative Examples 1 to 5 and a reference drug, in Experimental Example 2.
FIG. 8 shows the results of an experiment comparing the amount of dapagliflozin impurity (at RRT 0.74) generated under initial, accelerated or stress conditions between combination formulations of Examples 1 to 3 and Comparative Examples 1 to 5 and a reference drug, in Experimental Example 2.
FIG. 9 shows the results of an experiment comparing the amount of dapagliflozin impurity (at RRT 1.59) generated under initial, accelerated or stress conditions between combination formulations of Examples 1 to 3 and Comparative Examples 1 to 5 and a reference drug, in Experimental Example 2.

### DETAILED DESCRIPTION

In accordance with one embodiment of the present invention, the present invention is directed to a pharmaceutical combination formulation comprising: (1) a first release portion comprising sacubitril-valsartan or pharmaceutically acceptable salts thereof as an active ingredient; and (2) a second release portion comprising a sodium-glucose cotransporter-2 (SGLT-2) inhibitor or a pharmaceutically acceptable salt thereof as an active ingredient.

In the combination formulation according to the present invention, the first release portion and the second release portion may be present in a state in which they are physically separated from each other.

In the present invention, the combination formulation may be in the form of a bilayer tablet or multilayer tablet, or may be in the form of a drug-coated tablet having a core-shell structure.

In the bilayer tablet or multilayer tablet of the present invention, the first layer may comprise the first release portion or the second release portion, and the second layer may comprise the second release portion or the first release portion.

In the present invention, the drug-coated tablet may be composed of a structure comprising: a core; and a coating layer with which at least a portion of the surface of the core is coated. In the present invention, the core may comprise the first release portion or the second release portion, and the coating layer may comprise the second release portion or the first release portion.

In an example, the first layer may comprise an SGLT-2 inhibitor or a pharmaceutically acceptable salt thereof, and the second layer may comprise sacubitril-valsartan or pharmaceutically acceptable salts thereof.

In another example, the combination formulation may be a bilayer tablet comprising: a first layer comprising sacubitril-valsartan or pharmaceutically acceptable salts thereof; and a second layer comprising an SGLT-2 inhibitor, such as dapagliflozin, or a pharmaceutically acceptable salt thereof.

In the drug-coated tablet of the present invention, the core may be composed of the first release portion, and the drug coating layer may be composed of the second release portion.

In an example, the core may comprise sacubitril-valsartan or pharmaceutically acceptable salts thereof, and the coating layer may comprise an SGLT-2 inhibitor or a pharmaceutically acceptable salt thereof.

In the present invention, the first release portion and the second release portion may further comprise pharmaceutically acceptable additives such as an excipient (diluent), a disintegrant, a lubricant, or a coating agent, depending on the form of the combination formulation.

In the present invention, the combination formulation may be a bilayer tablet or a multilayer tablet, and the first or second release portion may comprise at least one pharmaceutically acceptable additive selected from the group consisting of an excipient (diluent), a disintegrant, and a lubricant.

In an example, the combination formulation may be a bilayer tablet or a multilayer tablet, the first release portion may comprise an excipient (diluent), a disintegrant, and a lubricant, in addition to the active ingredient, and the second release portion may also comprise an excipient (diluent), a disintegrant, and a lubricant, in addition to the active ingredient.

In addition, in the present invention, the combination formulation may be a drug-coated tablet, and the first release portion or the second release portion may comprise a coating agent.

In an example, the combination formulation may be a drug-coated tablet, the first release portion may comprise, in addition to the active ingredient, at least one pharmaceutically acceptable additive selected from the group consisting of an excipient (diluent), a disintegrant, and a lubricant, and the second release portion may comprise the active ingredient and a coating agent.

In another example, the combination formulation may be a drug-coated tablet, the second release portion may comprise, in addition to the active ingredient, at least one pharmaceutically acceptable additive selected from the group consisting of an excipient (diluent), a disintegrant, and a lubricant, and the first release portion may comprise the active ingredient and a coating agent.

### Mode for Invention

In accordance with one embodiment of the present invention, the present invention is directed to a pharmaceutical combination formulation comprising: (1) a first release portion comprising sacubitril-valsartan or pharmaceutically acceptable salts thereof as an active ingredient; and (2) a second release portion comprising a sodium-glucose cotransporter-2 (SGLT-2) inhibitor or a pharmaceutically acceptable salt thereof as an active ingredient.

In the present invention, "sacubitril" is a compound of 4-{[(2S,4R)-1-(4-biphenylyl)-5-ethoxy-4-methyl-5-oxo-2-pentanyl]amino}-4-oxobutanoic acid represented by Formula 1 below. Sacubitril is a neprilysin (NEP) inhibitor, which is known to be activated to sacubitrilat by de-ethylation via esterases and act to degrade atrial and brain natriuretic peptides, which are blood pressure lowering peptides that reduce blood flow.

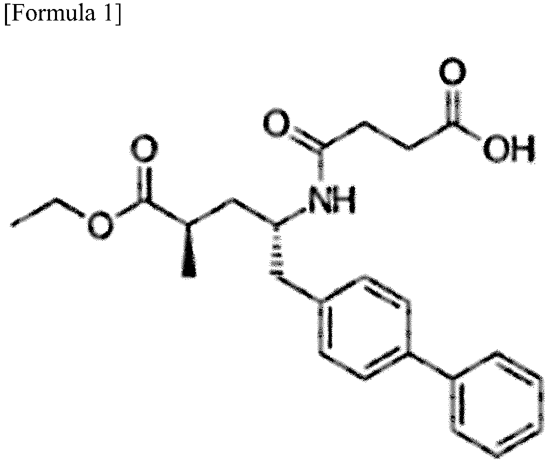

In the present invention, "valsartan" is a compound of (S)-N-(1-carboxy-2-methyl-prop-l-yl)-N-pentanoyl-N-[2'-(1H-tetrazol-5-yl)biphenyl-4-yl-methyl]amine represented by Formula 2 below. Valsartan is an angiotensin-II receptor blocker, which not only functions to block the action of angiotensin-II and relax blood vessels, thus lowering blood pressure, but is also used to treat heart failure, ischemic heart disease, etc.

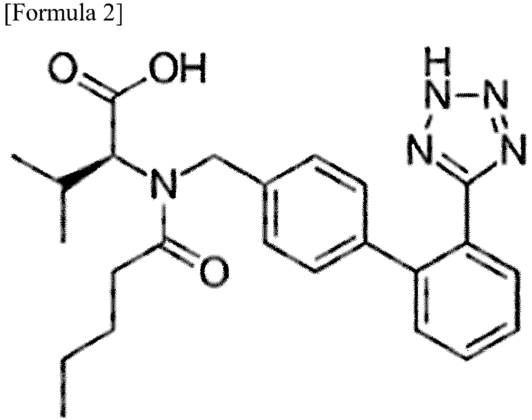

In the present invention, the "pharmaceutically acceptable salt" refers to salts commonly used in the art, and is meant to include salts formed with inorganic ions, inorganic acids, or organic acids, as well as hydrates or solvates of the salts. Examples of sacubitril-valsartan or pharmaceutically acceptable salts thereof suitable for the present invention include free acid forms, or salts of 1 to 3 inorganic ions such as sodium, calcium, potassium, or magnesium ions, or monohydrates to trihydrates thereof.

In the first release portion of the present invention, sacubitril or a pharmaceutically acceptable salt thereof and valsartan or a pharmaceutically acceptable salt thereof may be contained at a molar ratio of 1:5 to 5:1, preferably 1:1, without being limited thereto.

"Sacubitril-valsartan", which is contained as an active ingredient in the pharmaceutical combination formulation of the present invention, may be contained in the form of a supramolecular complex of sacubitril and valsartan, or may be contained in the form of a physical mixture of the free acids or salts of sacubitril and valsartan, or forms of these drugs include all of co-precipitation, co-crystal, or co-amorphous compounds, or single compounds linked by ionic bonds, non-ionic bonds, covalent bonds, non-covalent bonds, or hydrogen bonds. Here, the "supramolecular complex" is intended to describe an interaction between the two pharmaceutically active agents, the cations and any other entity present such as a solvent, in particular water, by means of noncovalent, intermolecular bonding between them. This interaction leads to an association of the species present in the supramolecular complex distinguishing this complex over a physical mixture of the species.

In an example, sacubitril-valsartan may be provided in the form of a compound represented by Formula 3 below, without being limited thereto.

[Formula 3] **[(A₁)(A₂)](Na⁺)₃ • x H₂O**

wherein
A₁ is valsartan in the dianionic form;
A₂ is sacubitril in the anionic form;
Na⁺ is a sodium ion; and
x may be a rational number ranging from 0.5 to 7, preferably a rational number from 2.5 to 3.5, more preferably 2.5 or 3, without being limited thereto.

In another example, sacubitril-valsartan may be a supramolecular complex of sacubitril and valsartan as trisodium salt trihydrate, without being limited thereto.

In another example, sacubitril-valsartan may be crystalline form II of sacubitril-valsartan trisodium trihydrate represented by Formula 4 below, without being limited thereto:

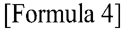

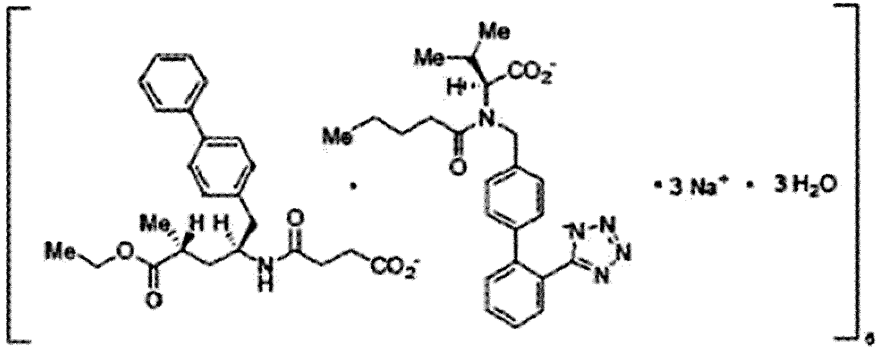

In another example, sacubitril-valsartan may be a supramolecular complex of sacubitril and valsartan as trisodium hemipentahydrate (see Korean Patent No. 10-1589317), conventionally referred to as "LCZ696", without being limited thereto.

In the present invention, sacubitril or N-(3-carboxy-1-oxopropyl)-(4S)-(p-phenylphenylmethyl)-4-amino-2R-methylbutanoic acid ethyl ester or a pharmaceutically acceptable salt thereof, as well as (2R,4S)-5-biphenyl-4-yl-4(3-carboxy-propionyl amino)-2-methyl-pentanoic acid, may be prepared by known methods such as the method described in U.S. Pat. No. 5,217,996, which is incorporated herein by reference.

In the present invention, valsartan or (S)-N-valeryl-N-{[2'-(1H-tetrazol-5-yl)-biphenyl-4-yl]-methyl}-valine) or a pharmaceutically acceptable salt thereof may be purchased from commercial sources or may be prepared according to known methods, such as the methods described in U.S. Patent No. 5,399,578 and European Patent No. 0443983, the preparation teachings of which are incorporated herein by reference. Valsartan may be used in certain embodiments of the invention in its free acid form, as well as in any suitable salt form. Depending on the circumstance, esters or other derivatives of the carboxylic grouping may be used, as well as salts and derivatives of the tetrazole grouping.

In the present invention, treatment with sacubitril-valsartan may be initiated at a starting dose of 50 mg (24.3/25.7 mg as sacubitril-valsartan) twice a day, and if necessary, the dose may be increased to 200 mg (97.2/102.8 mg as sacubitril-valsartan) twice a day.

In the pharmaceutical combination formulation of the present invention, the second release portion comprises an SGLT-2 inhibitor or a pharmaceutically acceptable salt thereof.

In the present invention, the SGLT-2 inhibitor is a glucopyranosyl-substituted benzene derivative compound having the ability to inhibit sodium-glucose cotransporters. In the present invention, the SGLT-2 inhibitor may be selected from the group consisting of dapagliflozin, empagliflozin, canagliflozin, ertugliflozin, sotagliflozin, ipragliflozin, tofogliflozin, luseogliflozin, bexagliflozin, and remogliflozin, without being limited thereto.

In an example, the SGLT-2 inhibitor may be dapagliflozin, without being limited thereto.

In another example, the SGLT-2 inhibitor may be empagliflozin, without being limited thereto.

In another example, the SGLT-2 inhibitor may be canagliflozin, without being limited thereto.

In another example, the SGLT-2 inhibitor may be ertugliflozin, without being limited thereto.

In another example, the SGLT-2 inhibitor may be sotagliflozin, without being limited thereto.

In another example, the SGLT-2 inhibitor may be ipragliflozin, without being limited thereto.

In another example, the SGLT-2 inhibitor may be tofogliflozin, without being limited thereto.

In another example, the SGLT-2 inhibitor may be luseogliflozin, without being limited thereto.

In another example, the SGLT-2 inhibitor may be bexagliflozin, without being limited thereto.

In another example, the SGLT-2 inhibitor may be remogliflozin, without being limited thereto.

In the present invention, the SGLT-2 inhibitor may be prepared according to methods known in the art, such as those shown in International Patent Publication No. WO 2006/120208, International Patent Publication No. WO 2007/031548, or International Patent Publication No. WO 2008/002824.

In the present invention, dapagliflozin is (1S)-1,5-andhydro-1-C-4-chloro-3-[(4-ethoxyphenyl)methyl]phenyl-D-glucitol represented by Formula 5 below. Dapagliflozin is able to inhibit SGLT-2 in the renal tubules and inhibit the glucose reabsorption process to excrete glucose into the urine, thereby suppressing the rise in blood glucose levels.

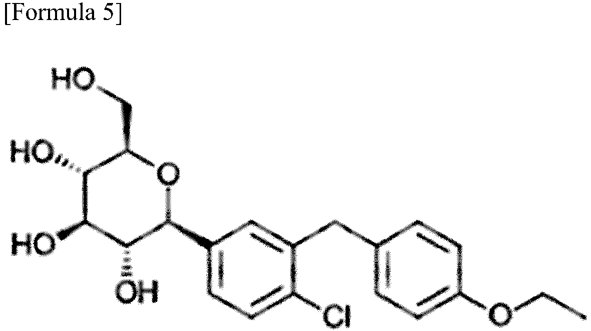

In the present invention, the SGLT-2 inhibitor, such as dapagliflozin, may include any pharmaceutically acceptable salt, ester, or solvate form thereof.

In the present invention, dapagliflozin or a pharmaceutically acceptable salt thereof may be in continuous and/or discontinuous amorphous form or in the form of a co-crystal.

In the present invention, dapagliflozin may include a prodrug ester thereof. Here, the pharmaceutically-acceptable ester may be cleaved in the human or animal body to produce the parent acid (e.g., where said ester is methoxymethyl) or hydroxy group (e.g., where said ester is an acetyl ester).

In the present invention, the solvate of dapagliflozin may comprise or consist of a propylene glycol solvate of dapagliflozin, such as dapagliflozin propylene glycol (1:1). In an example, the solvate of dapagliflozin may be in the form of its propylene glycol solvate hydrate (1:1:1). Here, the propylene glycol may be in the (S) form, the (R) form, or a mixture thereof. Preferably, the propylene glycol may be in the (S) form, without being limited thereto.

In addition, in the present invention, the solvate of dapagliflozin may be in the form of a solvate comprising dapagliflozin and 1,2-alkanediol or a hydrate thereof. Here, the 1,2-alkanediol may be 1,2-propanediol, 1,2-butanediol, 1,2-pentanediol, 1,2-hexanediol, and 1,2-heptanediol or a hydrate or mixture thereof. In an example, the solvate of dapagliflozin may be dapagliflozin propanediol monohydrate, or dapagliflozin compounded with (2S)-1,2-propanediol and hydrate at a ratio of about 1:1:1, without being limited thereto.

The second release portion of the present invention may comprise at least one amino acid together with dapagliflozin. Here, the amino acid may be selected from the group consisting of glycine, alanine, valine, leucine, isoleucine, proline, phenylalanine, methionine, tryptophan, serine, cysteine, threonine, tyrosine, asparagine, glutamine, aspartic acid, glutamic acid, arginine, histidine, and lysine. In addition, in the present invention, dapagliflozin and the amino acid may be in the form of a co-crystal. In an example, the second release portion may comprise dapagliflozin and proline (e.g., L-proline or D-proline), without being limited thereto.

The combination formulation of the present invention may contain, per dosage unit, 50 to 200 mg of sacubitril-valsartan as a sacubitril-valsartan free base and 5 to 10 mg of dapagliflozin as a free base. For example, the combination formulation may contain, per dosage unit, 100 mg or 200 mg of sacubitril-valsartan as a free base and 10 mg of dapagliflozin.

In the present specification, it is to be understood that, even if sacubitril, valsartan, the SGLT-2 inhibitor, or dapagliflozin is mentioned, it is meant to include all the above-mentioned salts, solvates and isomers thereof.

In the combination formulation according to the present invention, the first release portion and the second release portion may be present in a state in which they are physically separated from each other.

In the present invention, the combination formulation may be in the form of a bilayer tablet or multi-layer tablet, or may be in the form of a drug-coated tablet having a core-shell structure.

In the bilayer tablet or multilayer tablet of the present invention, the first layer may comprise the first release portion or the second release portion, and the second layer may comprise the second release portion or the first release portion.

In the bilayer tablet or multi-layer tablet of the present invention, the first layer may be composed of the first release portion, and the second layer may be composed of the second release portion.

In an example, the first layer may comprise sacubitril-valsartan or pharmaceutically acceptable salts thereof, and the second layer may comprise an SGLT-2 inhibitor or a pharmaceutically acceptable salt thereof.

In another example, the first layer may comprise an SGLT-2 inhibitor or a pharmaceutically acceptable salt thereof, and the second layer may comprise sacubitril-valsartan or a pharmaceutically acceptable salt thereof.

In another example, the combination formulation may be a bilayer tablet comprising: a first layer comprising sacubitril-valsartan or pharmaceutically acceptable salts thereof; and a second layer comprising an SGLT-2 inhibitor, such as dapagliflozin, or a pharmaceutically acceptable salt thereof. Here, the terms "first layer" and "second layer" are used to distinguish between different layers (i.e., the term "first layer" refers to any one layer of the bilayer tablet, and the term "second layer" refers to a different layer which is not the any one layer), and the first layer may be an upper layer or a lower layer, and the second layer may be a lower layer or an upper layer. These terms mean that sacubitril-valsartan and the SGLT-2 inhibitor are contained in different layers of this bilayer tablet.

In the present invention, the drug-coated tablet may be composed of a structure comprising: a core; and a coating layer with which at least a portion of the surface of the core is coated. In the present invention, the core may comprise the first release portion or the second release portion, and the coating layer may comprise the second release portion or the first release portion.

In the drug-coated tablet of the present invention, the core may be composed of the first release portion, and the drug coating layer may be composed of the second release portion.

In an example, the core may comprise sacubitril-valsartan or pharmaceutically acceptable salts thereof, and the coating layer may comprise an SGLT-2 inhibitor or a pharmaceutically acceptable salt thereof.

In another example, the core may comprise an SGLT-2 inhibitor or a pharmaceutically acceptable salt thereof, and the coating layer may comprise sacubitril-valsartan or pharmaceutically acceptable salts thereof.

In another example, the combination formulation may be a drug-coated tablet comprising: a core comprising sacubitril-valsartan or pharmaceutically acceptable salts thereof; and a coating layer comprising an SGLT-2 inhibitor, such as dapagliflozin, or a pharmaceutically acceptable salt thereof.

In the present invention, the first release portion and the second release portion may further comprise pharmaceutically acceptable additives such as an excipient (diluent), a disintegrant, a lubricant, or a coating agent, depending on the form of the combination formulation.

In the present invention, the combination formulation may be a bilayer tablet or a multi-layer tablet, and the first or second release portion may comprise at least one pharmaceutically acceptable additive selected from the group consisting of an excipient (diluent), a disintegrant, and a lubricant.

In addition, in the present invention, the combination formulation may be a drug-coated tablet, and the first release portion or the second release portion may comprise a coating agent.

In an example, the first release portion may comprise, in addition to the active ingredient, at least one pharmaceutically acceptable additive selected from the group consisting of an excipient (diluent), a disintegrant, and a lubricant, and the second release portion may comprise the active ingredient and a coating agent.

In another example, the second release portion may comprise, in addition to the active ingredient, at least one pharmaceutically acceptable additive selected from the group consisting of an excipient (diluent), a disintegrant, and a lubricant, and the first release portion may comprise the active ingredient and a coating agent.

In the present invention, sacubitril-valsartan comprised as an active ingredient in the first release portion may be comprised in an amount of 10 to 80 wt%, 20 to 80 wt%, or 30 to 80 wt%, based on the total weight of the first release portion, without being limited thereto.

In the present invention, the SGLT-2 inhibitor comprised as an active ingredient in the second layer, preferably dapagliflozin, may be comprised in an amount of 0.5 to 50 wt%, 0.5 to 30 wt%, or 0.5 to 10 wt%, based on the total weight of the second layer, without being limited thereto.

In the present invention, the excipient comprised in the first release portion may be comprised in an amount of 1 to 50 wt%, 5 to 50 wt%, 5 to 30 wt%, 10 to 30 wt%, 5 to 20 wt%, 10 to 20 wt%, or 5 to 15 wt%, based on the total weight of the first release portion, without being limited thereto.

In the present invention, the excipient comprised in the second release portion may be comprised in an amount of 20 to 95 wt%, 30 to 90 wt%, 40 to 90 wt%, 50 to 80 wt%, or 60 to 80 wt%, based on the total weight of the second release portion, without being limited thereto.

In the present invention, the disintegrant comprised in the first release portion may be comprised in an amount of 5 to 50 wt%, 10 to 50 wt%, 10 to 40 wt%, or 15 to 30 wt%, based on the total weight of the first release portion, without being limited thereto.

In the present invention, the disintegrant comprised in the second release portion may be comprised in an amount of 0.1 to 40 wt%, 0.1 to 20 wt%, 0.5 to 40 wt%, 0.5 to 20 wt%, 1 to 40 wt%, 5 to 40 wt%, 5 to 30 wt%, 10 to 30 wt%, 1 to 10 wt%, or 1 to 5 wt%, based on the total weight of the second release portion, without being limited thereto.

In the present invention, the lubricant comprised in the first release portion may be comprised in an amount of 0.1 to 20 wt%, 0.5 to 20 wt%, 1 to 10 wt%, or 2 to 8 wt%, based on the total weight of the first release portion, without being limited thereto.

In the present invention, the lubricant comprised in the second release portion may be comprised in an amount of 0.1 to 20 wt%, 0.5 to 20 wt%, 1 to 15 wt%, 5 to 15 wt%, 1 to 10 wt%, 5 to 10 wt%, 2 to 8 wt% or 5 to 8 wt%, based on the total weight of the second release portion, without being limited thereto.

In the present invention, the coating agent may be comprised in an amount of 30 to 90 wt%, 40 to 90 wt%, 40 to 80 wt%, or 50 to 80 wt%, based on the total weight of the first release portion or the second release portion, without being limited thereto.

In an example, the first release portion may comprise, based on the total weight of the first release portion, 10 to 80 wt% of the active ingredient, 1 to 50 wt% of the excipient, 5 to 50 wt% of the disintegrant, and 0.1 to 20 wt% of the lubricant, without being limited thereto.

In an example, the second release portion may comprise, based on the total weight of the second release portion, 0.5 to 50 wt% of the active ingredient, 20 to 95 wt% of the excipient, 0.1 to 20 wt% of the disintegrant, and 0.1 to 20 wt% of the lubricant, without being limited thereto.

In an example, the first release portion or the second release portion may comprise, based on the total weight thereof, 10 to 70 wt% of the active ingredient and 30 to 90 wt% of the coating agent, without being limited thereto.

In the present invention, the excipient (diluent) may be, for example, selected from the group consisting of microcrystalline cellulose, silicified microcrystalline cellulose, magnesium aluminometasilicate, magnesium aluminate silicate, aluminum silicate, sodium silicate, potassium silicate, magnesium silicate, calcium silicate, lactose, lactose hydrate, lactose anhydride, calcium hydrogen phosphate, hydroxypropyl cellulose, hydroxypropyl methylcellulose, dextrin, mannitol, sorbitol, starch, calcium phosphate monohydrate, calcium carbonate, sugars, and any mixtures thereof, without being limited thereto. However, in the present invention, anhydrous calcium phosphate has poor compatibility with dapagliflozin and sacubitril-valsartan, and thus is not included as the excipient.

In the present invention, the disintegrant may be, for example, selected from the group consisting of crospovidone, methylcellulose, cross-linked carboxymethylcellulose sodium (cross-linked CMC Na, C.CMC Na, or croscarmellose sodium), carboxymethylcellulose calcium, sodium starch glycolate, hydroxypropylcellulose, low-substituted hydroxypropylcellulose (L-HPC), hydroxypropyl methylcellulose, starch, pregelatinized starch, corn starch, potato starch, alginic acid or its sodium salt, and any combinations thereof, without being limited thereto.

In the present invention, the lubricant may be, for example, selected from the group consisting of calcium stearate, colloidal silicon dioxide (fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combinations thereof, without being limited thereto.

In the present invention, the coating agent is a hydrophilic polymer and may be, for example, at least one selected from the group consisting of hydroxypropyl methylcellulose (HPMC), polyvinyl alcohol (PVA), macrogol polyvinyl alcohol grafted copolymers, polymers of acrylic acid and its salts, polymethacrylate, poly(butylmethacrylate, 2-dimethylaminoethyl methacrylate, methyl methacrylate) copolymers (e.g., Eudragit^{®} E, Evonik), carboxymethylcellulose (sodium salt and calcium salt), ethylcellulose, methylcellulose, hydroxyethylcellulose, ethyl hydroxyethyl cellulose, hydroxypropylcellulose (HPC), L-HPC (low-substituted HPC), polyvinylpyrrolidone (povidone; PVP), vinylpyrrolidone-vinylacetate copolymers (e.g., Kollidon^{®} VA64, BASF), gelatin, guar gum, partially hydrolyzed starch, alginate, xanthan, and mixtures thereof, without being limited thereto. Preferably, the coating agent may be hydroxypropyl methylcellulose (HPMC), polyvinyl alcohol (PVA), a macrogol polyvinyl alcohol grafted copolymer, or a poly(butylmethacrylate, 2-dimethylaminoethyl methacrylate, methyl methacrylate) copolymer (e.g., Eudragit^{®} E, Evonik).

In the present invention, the first release portion may comprise an additive selected from among microcrystalline cellulose, mannitol, low-substituted hydroxypropyl cellulose (L-HPC), croscarmellose sodium, sodium starch glycolate, colloidal silicon dioxide (fumed silica, Aerosil), talc, magnesium stearate, and any combinations thereof.

In one example, the first release portion may comprise additives including: at least one of microcrystalline cellulose and mannitol; low-substituted hydroxypropylcellulose (L-HPC); at least one of croscarmellose sodium and sodium starch glycolate; colloidal silicon dioxide (fumed silica, Aerosil); talc; and magnesium stearate.

In another example, the first release portion may comprise additives including microcrystalline cellulose, mannitol, low-substituted hydroxypropylcellulose (L-HPC), croscarmellose sodium, colloidal silicon dioxide (fumed silica, Aerosil), talc, and magnesium stearate.

In another example, the first release portion may comprise additives including microcrystalline cellulose, low-substituted hydroxypropylcellulose (L-HPC), sodium starch glycolate, colloidal silicon dioxide (fumed silica, Aerosil), talc, and magnesium stearate.

In the present invention, the first release portion may comprise at least one of microcrystalline cellulose and mannitol in an amount of 1 to 50 wt%, 5 to 50 wt%, 1 to 30 wt%, 5 to 30 wt%, 1 to 20 wt%, or 5 to 20 wt%, based on the total weight of the first release portion. In an example, the first release portion may comprise microcrystalline cellulose. In another example, the first release portion comprise may comprise mannitol. In another example, the first release portion may comprise both microcrystalline cellulose and mannitol. In another example, the first release portion may comprise microcrystalline cellulose in an amount of 1 to 30 wt%, 1 to 20 wt%, or 1 to 10 wt%, without being limited thereto. In another example, the first release portion may comprise mannitol in an amount of 1 to 30 wt%, 1 to 20 wt%, or 1 to 10 wt%, without being limited thereto.

In the present invention, the first release portion may comprise low-substituted hydroxypropyl cellulose in an amount of 5 to 30 wt%, 5 to 20 wt%, 5 to 15 wt%, or 10 to 20 wt%, based on the total weight of the first release portion.

In the present invention, the first release portion may comprise at least one of sodium starch glycolate and croscarmellose sodium in an amount of 1 to 30 wt%, 5 to 30 wt%, 5 to 20 wt%, or 5 to 15 wt%, based on the total weight of the first release portion. In an example, the first release portion may comprise sodium starch glycolate. In another example, the first release portion may comprise croscarmellose sodium. In another example, the first release portion may comprise both sodium starch glycolate and croscarmellose sodium. In another example, the first release portion may comprise sodium starch glycolate in an amount of 1 to 30 wt%, 5 to 30 wt%, 5 to 20 wt%, or 5 to 15 wt%, without being limited thereto. In another example, the first release portion may comprise croscarmellose sodium in an amount of 1 to 30 wt%, 5 to 30 wt%, 5 to 20 wt%, or 5 to 15 wt%, without being limited thereto.

In the present invention, the first release portion may comprise colloidal silicon dioxide in an amount of 0.1 to 10 wt%, 0.1 to 5 wt%, 0.1 to 3 wt%, or 0.1 to 2 wt%, based on the total weight of the first release portion.

In the present invention, the first release portion may comprise talc in an amount of 0.1 to 10 wt%, 0.1 to 5 wt%, 0.1 to 3 wt%, or 0.1 to 2 wt%, based on the total weight of the first release portion.

In the present invention, the first release portion may comprise magnesium stearate in an amount of 0.1 to 10 wt%, 0.1 to 5 wt%, 1 to 5 wt%, or 1 to 3 wt%, based on the total weight of the first release portion.

In one example, the first release portion may comprise, based on the total weight of the first release portion, 10 to 80 wt% of the active ingredient, 5 to 50 wt% of at least one of microcrystalline cellulose and mannitol, 5 to 30 wt% of low-substituted hydroxypropyl cellulose, 5 to 30 wt% of at least one of sodium starch glycolate and croscarmellose sodium, 0.1 to 10 wt% of colloidal silicon dioxide, 0.1 to 10 wt% of talc, and 0.1 to 10 wt% of magnesium stearate, without being limited thereto.

In another example, the first release portion may comprise, based on the total weight of the first release portion, 10 to 80 wt% of active ingredient, 5 to 50 wt% of microcrystalline cellulose, 5 to 30 wt% of low-substituted hydroxypropyl cellulose, 5 to 30 wt% of sodium starch glycolate, 0.1 to 10 wt% of colloidal silicon dioxide, 0.1 to 10 wt% of talc, and 0.1 to 10 wt% of magnesium stearate, without being limited thereto.

In another example, the first release portion may comprise, based on the total weight of the first release portion, 10 to 80 wt% of active ingredient, 1 to 30 wt% of microcrystalline cellulose, 1 to 20 wt% of mannitol, 5 to 30 wt% of low-substituted hydroxypropyl cellulose, 5 to 30 wt% of croscarmellose sodium, 0.1 to 10 wt% of colloidal silicon dioxide, 0.1 to 10 wt% of talc, and 0.1 to 10 wt% of magnesium stearate, without being limited thereto.

In another example, the first release portion may comprise, based on the total weight of the first release portion, 10 to 80 wt% of active ingredient, 1 to 10 wt% of microcrystalline cellulose, 1 to 10 wt% of mannitol, 5 to 20 wt% of low-substituted hydroxypropyl cellulose, 5 to 20 wt% of croscarmellose sodium, 0.1 to 10 wt% of colloidal silicon dioxide, 0.1 to 10 wt% of talc, and 0.1 to 10 wt% of magnesium stearate, without being limited thereto.

In the present invention, the second release portion may comprise an additive selected from among microcrystalline cellulose, lactose hydrate, mannitol, hydroxypropyl cellulose, sodium stearyl fumarate, croscarmellose sodium, and any combinations thereof.

In one example, the second release portion may comprise additives including: at least one of microcrystalline cellulose and lactose hydrate; mannitol; hydroxypropylcellulose; sodium stearyl fumarate; and croscarmellose sodium.

In another example, the second release portion may comprise additives including microcrystalline cellulose, mannitol, hydroxypropyl cellulose, sodium stearyl fumarate, and croscarmellose sodium.

In another example, the second release portion may comprise additives including lactose hydrate, mannitol, hydroxypropylcellulose, sodium stearyl fumarate, and croscarmellose sodium.

In the present invention, the second release portion may comprise at least one of microcrystalline cellulose and lactose hydrate in an amount of 10 to 50 wt%, 20 to 50 wt%, 10 to 40 wt%, 15 to 40 wt%, 10 to 30 wt%, or 15 to 30 wt%, based on the total weight of the second release portion. In an example, the second release portion may comprise microcrystalline cellulose. In another example, the second release portion may comprise lactose hydrate. In another example, the second release portion may comprise both microcrystalline cellulose and lactose hydrate. In another example, the second release portion may comprise microcrystalline cellulose in an amount of 10 to 50 wt%, 10 to 40 wt%, or 15 to 30 wt%. In another example, the second release portion may comprise lactose hydrate in an amount of 10 to 50 wt%, 20 to 50 wt%, 10 to 40 wt%, or 15 to 40 wt%, without being limited thereto.

In the present invention, the second release portion may comprise mannitol in an amount of 10 to 70 wt%, 20 to 70 wt%, 30 to 70 wt%, 40 to 70 wt%, 10 to 60 wt%, 20 to 60 wt%, 30 to 60 wt%, 40 to 60 wt%, 10 to 50 wt%, 20 to 50 wt%, or 30 to 50 wt%, based on the total weight of the second release portion.

In the present invention, the second release portion may comprise hydroxypropyl cellulose in an amount of 0.1 to 20 wt%, 0.5 to 20 wt%, 0.1 to 10 wt%, 0.5 to 10 wt%, 1 to 10 wt%, or 2 to 8 wt%, based on the total weight of the second release portion.

In the present invention, the second release portion may comprise croscarmellose sodium in an amount of 0.5 to 20 wt%, 1 to 20 wt%, 5 to 20 wt%, or 5 to 15 wt%, based on the total weight of the second release portion.

In the present invention, the second release portion may comprise sodium stearyl fumarate in an amount of 0.5 to 20 wt%, 1 to 20 wt%, 5 to 20 wt%, or 5 to 15 wt%, based on the total weight of the second release portion.

In one example, the second release portion may comprise, based on the total weight of the second release portion, 0.5 to 50 wt% of the active ingredient, 10 to 50 wt% of at least one of microcrystalline cellulose and lactose hydrate, 10 to 50 wt% of mannitol, 0.1 to 20 wt% of hydroxypropyl cellulose, 0.5 to 20 wt% of croscarmellose sodium, and 0.5 to 20 wt% of sodium stearyl fumarate, without being limited thereto.

In another example, the second release portion may comprise, based on the total weight of the second release portion, 0.5 to 50 wt% of the active ingredient, 10 to 50 wt% of microcrystalline cellulose, 10 to 50 wt% of mannitol, 0.1 to 20 wt% of hydroxypropyl cellulose, 0.5 to 20 wt% of croscarmellose sodium, and 0.5 to 20 wt% of sodium stearyl fumarate, without being limited thereto.

In another example, the second release portion may comprise, based on the total weight of the second release portion, 0.5 to 50 wt% of the active ingredient, 10 to 30 wt% of microcrystalline cellulose, 20 to 70 wt% of mannitol, 0.5 to 20 wt% of hydroxypropyl cellulose, 0.5 to 20 wt% of croscarmellose sodium, and 0.1 to 20 wt% of sodium stearyl fumarate, without being limited thereto.

In another example, the second release portion may comprise, based on the total weight of the second release portion, 0.5 to 50 wt% of the active ingredient, 10 to 50 wt% of lactose hydrate, 10 to 50 wt% of mannitol, 0.1 to 20 wt% of hydroxypropyl cellulose, 0.5 to 20 wt% of croscarmellose sodium, and 0.5 to 20 wt% of sodium stearyl fumarate, without being limited thereto.

In addition, in the present invention, the first release portion may comprise polyvinylpyrrolidone (povidone; PVP) and a macrogol-polyvinyl alcohol copolymer, in addition to the active ingredient.

In the present invention, the first release portion may comprise polyvinylpyrrolidone (povidone; PVP) in an amount of 1 to 20 wt%, 5 to 20 wt%, 5 to 15 wt%, or 5 to 10 wt%, based on the total weight of the first release portion.

In the present invention, the first release portion may comprise a macrogol-polyvinyl alcohol copolymer in an amount of 30 to 80 wt%, 40 to 80 wt%, 30 to 70 wt%, or 40 to 70 wt%, based on the total weight of the first release portion.

In one example, the first release portion may comprise, based on the total weight of the first release portion, 10 to 70 wt% of the active ingredient, 5 to 20 wt% of povidone (PVP), and 30 to 80 wt% of a macrogol-polyvinyl alcohol copolymer, without being limited thereto.

In the present invention, the second release portion may comprise polyvinylpyrrolidone (povidone; PVP) and a macrogol-polyvinyl alcohol copolymer, in addition to the active ingredient.

In the present invention, the second release portion may comprise polyvinylpyrrolidone (povidone; PVP) in an amount of 1 to 20 wt%, 5 to 20 wt%, 5 to 15 wt%, or 5 to 10 wt%, based on the total weight of the second release portion.

In the present invention, the second release portion may comprise a macrogol-polyvinyl alcohol copolymer in an amount of 30 to 80 wt%, 40 to 80 wt%, 30 to 70 wt%, or 40 to 70 wt%, based on the total weight of the second release portion.

In one example, the second release portion may comprise, based on the total weight of the second release portion, 10 to 70 wt% of the active ingredient, 5 to 20 wt% of povidone (PVP), and 30 to 80 wt% of a macrogol-polyvinyl alcohol copolymer, without being limited thereto.

In the present invention, when the combination formulation is prepared in the form of a drug-coated tablet, the coating layer may further comprise various biologically inactive ingredients for additional purposes such as coating efficiency, drug stability, appearance, color, protection, maintenance, binding, performance improvement, and preparation process improvement. Biologically inactive ingredients that may be additionally contained in the coating layer may be one or more selected from the group consisting of plasticizers, lubricants, colorants, fragrances, surfactants, stabilizers, antioxidants, foaming agents, anti-foaming agents, paraffin, and waxes, without being limited thereto.

In addition, in the present invention, the first release portion and/or the second release portion may further contain various additives in order to improve the physical properties, manufacturability, compressibility, appearance, taste, and/or drug stability of the final combination formulation. These additives include, for example, stabilizers, solubilizers, sweeteners, flavor enhancers, pigments, wetting agents, fillers, stabilizers, surfactants, lubricants, solubilizers, buffers, sweeteners, adsorbents, flavor enhancers, binders, suspending agents, curing agents, antioxidants, pharmaceutical brighteners, fragrances, flavoring agents, pigments, coating agents, wetting agents, wetting regulators, anti-foaming agents, refreshing agents, masticating agents, anti-static agents, coloring agents, sugar coating agents, isotonic agents, softeners, emulsifiers, adhesives, thickeners, foaming agents, pH regulators, excipients, dispersants, disintegrants, water-proofing agents, preservatives, preserving agents, dissolution aids, solvents and fluidizing agents, but are not limited thereto and any pharmaceutically acceptable additives may be used.

In the present invention, in the combination formulation in the form of a bilayer tablet as described above, the tablet-shaped layer comprising each of the first and second release portions may comprise granules, containing the active ingredient, and a post-mixture portion.

In the present invention, the layer comprising the first release portion (first layer) may comprise granules, containing the active ingredient, and a post-mixture portion. Here, the granules may further contain pharmaceutically acceptable additives such as an excipient, a disintegrant, or a lubricant. Additionally, the post-mixture portion may further comprise at least one pharmaceutically acceptable additive selected from among a disintegrant and a lubricant.

In one example, the layer comprising the first release portion may comprise granules, which contain the active ingredient, an excipient, a disintegrant, and a lubricant, and a post-mixture portion which comprises a disintegrant and a lubricant, without being limited thereto.

In the present invention, the granules in the layer comprising the first release portion may contain the active ingredient in an amount of 10 to 80 wt%, 20 to 80 wt%, or 30 to 80 wt%, based on the total weight of the first release portion.

In the present invention, the granules in the layer comprising the first release portion may contain an excipient in an amount of 5 to 50 wt%, 5 to 30 wt%, or 5 to 20 wt%, based on the total weight of the first release portion.

In the present invention, the granules in the layer comprising the first release portion may contain a disintegrant in an amount of 1 to 30 wt%, 10 to 30 wt%, or 10 to 20 wt%, based on the total weight of the first release portion.

In the present invention, the granules in the layer comprising the first release portion may contain a lubricant in an amount of 0.1 to 10 wt%, 1 to 10 wt%, or 1 to 5 wt%, based on the total weight of the first release portion.

In the present invention, the post-mixture portion in the layer comprising the first release portion may comprise a disintegrant in an amount of 1 to 20 wt%, 1 to 10 wt%, or 2 to 8 wt%, based on the total weight of the first release portion.

In the present invention, the post-mixture portion in the layer comprising the first release portion may comprise a lubricant in an amount of 0.1 to 10 wt%, 0.1 to 5 wt%, or 1 to 5 wt%, based on the total weight of the first release portion.

In one example, the granules in the layer comprising the first release portion may comprise, based on the total weight of the first release portion, 10 to 80 wt% of the active ingredient, 5 to 50 wt% of an excipient, 1 to 30 wt% of a disintegrant, and 0.1 to 10 wt% of a lubricant, and the post-mixture portion may comprise, based on the total weight of the first release portion, 1 to 20 wt% of a disintegrant and 0.1 to 10 wt% of a lubricant, without being limited thereto.

In another example, the granules in the layer comprising the first release portion may comprise, based on the total weight of the first release portion, 10 to 80 wt% of the active ingredient, 5 to 50 wt% of an excipient, 1 to 30 wt% of a disintegrant, and 0.1 to 10 wt% of a lubricant, and the post-mixture portion may comprise, based on the total weight of the first release portion, 1 to 20 wt% of a disintegrant and 0.1 to 10 wt% of a lubricant, without being limited thereto.

In another example, the granules in the layer comprising the first release portion may comprise, based on the total weight of the first release portion, 10 to 80 wt% of the active ingredient, 5 to 20 wt% of an excipient, 10 to 30 wt% of a disintegrant, and 0.1 to 10 wt% of a lubricant, and the post-mixture portion may comprise, based on the total weight of the first release portion, 1 to 20 wt% of a disintegrant and 0.1 to 10 wt% of a lubricant, without being limited thereto.

In the present invention, the granules in the layer comprising the first release portion may comprise at least one of microcrystalline cellulose and mannitol in an amount of 1 to 50 wt%, 5 to 50 wt%, 1 to 30 wt%, 5 to 30 wt%, 1 to 20 wt%, or 5 to 20 wt%. In an example, the granules in the layer comprising the first release portion may comprise microcrystalline cellulose. In another example, the granules in the layer may comprise mannitol. In another example, the granules in the layer may comprise both microcrystalline cellulose and mannitol. In another example, the granules in the layer may comprise microcrystalline cellulose in an amount of 1 to 30 wt%, 1 to 20 wt%, or 1 to 10 wt%, without being limited thereto. In another example, the granules in the layer may comprise mannitol in an amount of 1 to 30 wt%, 1 to 20 wt%, or 1 to 10 wt%, without being limited thereto.

In the present invention, the granules in the layer comprising the first release portion may comprise low-substituted hydroxypropyl cellulose in an amount of 5 to 30 wt%, 5 to 20 wt%, 5 to 15 wt%, or 10 to 20 wt%.

In the present invention, the granules in the layer comprising the first release portion may comprise at least one of sodium starch glycolate and croscarmellose sodium in an amount of 1 to 15 wt%, 1 to 10 wt%, or 2 to 8 wt%. In an example, the granules in the layer comprising the first release portion may comprise sodium starch glycolate. In another example, the granules in the layer may comprise croscarmellose sodium. In another example, the granules in the layer may comprise both sodium starch glycolate and sodium croscarmellose. In another example, the granules in the layer may comprise sodium starch glycolate in an amount of 1 to 15 wt%, 1 to 10 wt%, or 2 to 8 wt%, without being limited thereto. In another example, the granules in the layer may comprise croscarmellose sodium in an amount of 1 to 15 wt%, 1 to 10 wt%, or 2 to 8 wt%, without being limited thereto.

In the present invention, the granules in the layer comprising the first release portion may comprise colloidal silicon dioxide in an amount of 0.1 to 5 wt%, 0.1 to 3 wt%, or 0.1 to 2 wt%.

In the present invention, the granules in the layer comprising the first release portion may comprise talc in an amount of 0.1 to 10 wt%, 0.1 to 5 wt%, or 1 to 5 wt%.

In the present invention, the granules in the layer comprising the first release portion may comprise magnesium stearate in an amount of 0.1 to 5 wt%, 0.5 to 5 wt%, or 1 to 5 wt%.

In one example, the granules in the layer comprising the first release portion may comprise, based on the total weight of the first release portion, 10 to 80 wt% of the active ingredient, 1 to 50 wt% of at least one of microcrystalline cellulose and mannitol, 5 to 30 wt% of low-substituted hydroxypropyl cellulose, 1 to 15 wt% of at least one of sodium starch glycolate and croscarmellose sodium, 0.1 to 5 wt% of colloidal silicon dioxide, 0.1 to 10 wt% of talc, and 0.1 to 5 wt% of magnesium stearate, and the post-mixture portion may comprise, based on the total weight of the first release portion, 1 to 15 wt% of sodium starch glycolate, 0.1 to 5 wt% of colloidal silicon dioxide, and 0.1 to 5 wt% of magnesium stearate, without being limited thereto.

In another example, the granules in the layer comprising the first release portion may comprise, based on the total weight of the first release portion, 10 to 80 wt% of the active ingredient, 5 to 50 wt% of microcrystalline cellulose, 5 to 30 wt% of low-substituted hydroxypropyl cellulose, 1 to 15 wt% of sodium starch glycolate, 0.1 to 5 wt% of colloidal silicon dioxide, 0.1 to 10 wt% of talc, and 0.1 to 5 wt% of magnesium stearate, and the post-mixture portion may comprise, based on the total weight of the first release portion, 1 to 15 wt% of sodium starch glycolate, 0.1 to 5 wt% of colloidal silicon dioxide, and 0.1 to 5 wt% of magnesium stearate, without being limited thereto.

In another embodiment, the granules in the layer comprising the first release portion may comprise, based on the total weight of the first release portion, 10 to 80 wt% of the active ingredient, 1 to 30 wt% of microcrystalline cellulose, 1 to 20 wt% of mannitol, 5 to 30 wt% of low-substituted hydroxypropyl cellulose, 1 to 15 wt% of croscarmellose sodium, 0.1 to 5 wt% of colloidal silicon dioxide, 0.1 to 10 wt% of talc, and 0.1 to 5 wt% of magnesium stearate, and the post-mixture portion may comprise, based on the total weight of the first layer, 1 to 15 wt% of croscarmellose sodium, 0.1 to 5 wt% of colloidal silicon dioxide, and 0.1 to 5 wt% of magnesium stearate, without being limited thereto.

In another embodiment, the granules in the layer comprising the first release portion may comprise, based on the total weight of the first release portion, 10 to 80 wt% of the active ingredient, 1 to 20 wt% of microcrystalline cellulose, 1 to 20 wt% of mannitol, 5 to 20 wt% of low-substituted hydroxypropyl cellulose, 1 to 10 wt% of croscarmellose sodium, 0.1 to 5 wt% of colloidal silicon dioxide, 0.1 to 5 wt% of talc, and 0.1 to 5 wt% of magnesium stearate, and the post-mixture portion may comprise, based on the total weight of the first layer, 1 to 10 wt% of croscarmellose sodium, 0.1 to 5 wt% of colloidal silicon dioxide, and 0.1 to 5 wt% of magnesium stearate, without being limited thereto.

In the present invention, the layer (second layer) comprising the second release portion may comprise granules, which comprise the active ingredient, and a post-mixture portion. Here, the granules may additionally comprise pharmaceutically acceptable additives such as an excipient, a disintegrant, or a lubricant. In addition, the post-mixture portion may further include at least one pharmaceutically acceptable additive selected from among a disintegrant and a lubricant.

In one example, the layer comprising the second release portion may comprise granules, comprising the active ingredient, an excipient, a disintegrant, and a lubricant, and a post-mixture portion comprising a disintegrant and a lubricant, without being limited thereto.

In the present invention, the granules in the layer comprising the second release portion may comprise the active ingredient in an amount of 0.5 to 50 wt%, 0.5 to 30 wt%, or 0.5 to 10 wt%, based on the total weight of the second release portion.

In the present invention, the granules in the layer comprising the second release portion may comprise an excipient in an amount of 20 to 95 wt%, 30 to 90 wt%, 40 to 90 wt%, 50 to 80 wt%, or 60 to 80 wt%, based on the total weight of the second release portion.

In the present invention, the granules in the layer comprising the second release portion may comprise a disintegrant in an amount of 0.1 to 20 wt%, 1 to 10 wt%, or 2 to 8 wt%, based on the total weight of the second release portion.

In the present invention, the granules in the layer comprising the second release portion may comprise a lubricant in an amount of 0.1 to 20 wt%, 1 to 10 wt%, or 2 to 8 wt%, based on the total weight of the second release portion.

In the present invention, the post-mixture portion in the layer comprising the second release portion may comprise a disintegrant in an amount of 1 to 30 wt%, 1 to 20 wt%, 5 to 20 wt%, or 5 to 10 wt%, based on the total weight of the second release portion.

In the present invention, the post-mixture portion in the layer comprising the second release portion may comprise a lubricant in an amount of 0.1 to 20 wt%, 1 to 10 wt%, or 2 to 8 wt%, based on the total weight of the second release portion.

In one example, the granules in the layer comprising the second release portion may comprise, based on the total weight of the second release portion, 0.5 to 50 wt% of the active ingredient, 20 to 95 wt% of an excipient, 0.1 to 20 wt% of a disintegrant, and 0.1 to 10 wt% of a lubricant, and the post-mixture portion may comprise, based on the total weight of the second release portion, 1 to 30 wt% of a disintegrant and 0.1 to 10 wt% of a lubricant, without being limited thereto.

In another example, the granules in the layer comprising the second release portion may comprise, based on the total weight of the second release portion, 0.5 to 50 wt% of the active ingredient, 50 to 90 wt% of an excipient, 1 to 10 wt% of a disintegrant, and 1 to 10 wt% of a lubricant, and the post-mixture portion may comprise, based on the total weight of the second release portion, 5 to 20 wt% of a disintegrant and 0.1 to 10 wt% of a lubricant, without being limited thereto.

In another example, the granules in the layer comprising the second release portion may comprise, based on the total weight of the second release portion, 0.5 to 50 wt% of the active ingredient, 10 to 50 wt% of at least one of microcrystalline cellulose and lactose hydrate, 10 to 50 wt% of mannitol, 0.1 to 20 wt% of hydroxypropyl cellulose, and 0.1 to 10 wt% of sodium stearyl fumarate, and the post-mixture portion may comprise, based on the total weight of the second release portion, 0.5 to 20 wt% of croscarmellose sodium and 0.1 to 10 wt% of sodium stearyl fumarate, without being limited thereto.

In another example, the granules in the layer comprising the second release portion may comprise, based on the total weight of the second release portion, 0.5 to 50 wt% of the active ingredient, 10 to 30 wt% of microcrystalline cellulose, 20 to 70 wt% of mannitol, 0.5 to 20 wt% of hydroxypropyl cellulose, and 0.1 to 10 wt% of sodium stearyl fumarate, and the post-mixture portion may comprise, based on the total weight of the second layer, 0.5 to 20 wt% of croscarmellose sodium and 0.1 to 10 wt% of sodium stearyl fumarate, without being limited thereto.

Since the combination formulation according to the present invention as described above contains both two pharmacologically active ingredients, that is, sacubitril-valsartan and an SGLT-2 inhibitor, such as dapagliflozin, in one dosage unit, it may be useful in the treatment or prevention of heart failure, especially heart failure that is not sufficiently controlled with a single drug, alleviates the inconvenience of having to take two medications separately, and significantly improves the patient's medication compliance. The combination formulation may be administered once a day, twice a day, three times a day, or four times a day, depending on the contents of the active ingredients contained therein.

In addition, since the combination formulation of the present invention is in the form of a double-layer tablet or drug-coated tablet in which the first release portion comprising sacubitril-valsartan and the second release portion comprising the SGLT-2 inhibitor are physically separated from each other, it enables the SGLT-2 inhibitor to dissolve at a faster rate than sacubitril-valsartan, so that sacubitril-valsartan, which has low solubility in a low-pH environment such as stomach acid, does not inhibit the dissolution of the SGLT-2 inhibitor, whereby all of these ingredients have an immediate-release dissolution pattern. Thus, the combination formulation has an advantage in that each of these ingredients can exhibit a quick and sufficient pharmacological effect within the administration period.

Furthermore, the combination formulation according to the present invention has a synergistic therapeutic effect due to the combined use of sacubitril-valsartan and the SGLT-2 inhibitor, and has high safety by suppressing the generation of impurities.

In one example, in the combination formulation according to the present invention, the dissolution rate (by weight) of the SGLT-2 inhibitor (e.g., dapagliflozin) during a dissolution test (pH 1.2 buffer, 37°C, 900 mL, 50 rpm) may be 50% or more, 60% or more, 70% or more, or 80% or more within 10 minutes, 80% or more, 85% or more, or 90% or more within 15 minutes, and 85% or more, 90% or more, or 95% or more within 30 minutes.

In another example, in the combination formulation according to the present invention, the dissolution rate of sacubitril during a dissolution test (pH 1.2 buffer, 37°C, 900 mL, 50 rpm) may be 10 to 40% after 30 minutes and 50% or less after 2 hours, without being limited thereto.

In another example, in the composite preparation according to the present invention, the dissolution rate of valsartan during a dissolution test (pH 1.2 buffer, 37°C, 900 mL, 50 rpm) may be 10 to 40% after 30 minutes and 50% or less after 2 hours, without being limited thereto.

In another example, in the combination formulation according to the present invention, the dissolution rate (by weight) of the SGLT-2 inhibitor (e.g., dapagliflozin) during a dissolution test (pH 6.8 buffer, 37°C, 900 mL, 50 rpm) may be 50 to 95%, or 60 to 90% within 10 minutes, 80% or more, 85% or more, or 90% or more within 15 minutes, and 90% or more, or 95% or more within 30 minutes.

In another example, in the combination formulation according to the present invention, the dissolution rate of sacubitril during a dissolution test (pH 6.8 buffer, 37°C, 900 mL, 50 rpm) may be 80% or more, 90% or more, or 95% or more within 30 minutes, without being limited thereto.

In another example, in the composite preparation according to the present invention, the dissolution rate of valsartan during a dissolution test (pH 6.8 buffer, 37°C, 900 mL, 50 rpm) may be 80% or more, 90% or more, or 95% or more within 30 minutes, without being limited thereto.

According to another embodiment of the present invention, the present invention relates to a method for preparing the combination formulation according to the present invention, the method comprising steps of:
(a) preparing a first release portion comprising sacubitril-valsartan or pharmaceutically acceptable salts thereof as an active ingredient;
(b) preparing a second release portion comprising an SGLT-2 inhibitor or a pharmaceutically acceptable salt thereof as an active ingredient; and
(c) formulating the first release portion and the second release portion into a single solid formulation.

In the present invention, in step (c), the combination formulation may be formulated in the form of a bilayer tablet or a multilayer tablet, or in the form of a drug-coated tablet having a core-shell structure.

In the present invention, steps (a) and (b) do not necessarily have to be performed sequentially, and step (b) may be performed after performing step (a), or step (a) may be performed after performing step (b), or steps (a) and (b) may be performed simultaneously.

In the present invention, step (a) of preparing the first release portion may comprise steps of: (a-1) mixing sacubitril-valsartan or pharmaceutically acceptable salts thereof and a pharmaceutically acceptable additive; (a-2) preparing granules; and (a-3) mixing the granules and a post-mixture portion.

In the present invention, in step (a-1), sacubitril or a pharmaceutically acceptable salt thereof and valsartan or a pharmaceutically acceptable salt thereof may be contained in the first release portion of the present invention at a molar ratio of 1:5 to 5:1, preferably 1:1, without being limited thereto.

In the present invention, the pharmaceutically acceptable additive in step (a-1) or the post-mixture portion in step (a-3) may each comprise at least one selected from the group consisting of an excipient, a disintegrant, and a lubricant.

In the present invention, the excipient (diluent) may be, for example, selected from the group consisting of microcrystalline cellulose, silicified microcrystalline cellulose, magnesium aluminometasilicate, magnesium aluminate silicate, aluminum silicate, sodium silicate, potassium silicate, magnesium silicate, calcium silicate, lactose, lactose hydrate, lactose anhydride, calcium hydrogen phosphate, hydroxypropyl cellulose, hydroxypropyl methylcellulose, dextrin, mannitol, sorbitol, starch, calcium phosphate monohydrate, calcium carbonate, sugars, and any mixtures thereof, without being limited thereto. However, in the present invention, anhydrous calcium phosphate has poor compatibility with dapagliflozin and sacubitril-valsartan, and thus is not included as the excipient (diluent).

In the present invention, the disintegrant may be, for example, selected from the group consisting of crospovidone, methylcellulose, cross-linked carboxymethylcellulose sodium (cross-linked CMC Na, C.CMC Na, or croscarmellose sodium), carboxymethylcellulose calcium, sodium starch glycolate, hydroxypropylcellulose, low-substituted hydroxypropylcellulose (L-HPC), hydroxypropyl methylcellulose, starch, pregelatinized starch, corn starch, potato starch, alginic acid or its sodium salt, and any combinations thereof, without being limited thereto.

In the present invention, the lubricant may be, for example, selected from the group consisting of calcium stearate, colloidal silicon dioxide (fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combinations thereof, without being limited thereto.

In an example, in step (a-1), sacubitril-valsartan or pharmaceutically acceptable salts thereof as an active ingredient, and a pharmaceutically acceptable additive selected from among microcrystalline cellulose, mannitol, low-substituted hydroxypropylcellulose (L-HPC), croscarmellose sodium, sodium starch glycolate, colloidal silicon dioxide (fumed silica, Aerosil), talc, magnesium stearate, and any combinations thereof may be adjusted to appropriate contents and mixed together to prepare granules.

In another example, the mixture obtained in step (a-1) may comprise sacubitril-valsartan or pharmaceutically acceptable salts thereof as an active ingredient, and a pharmaceutically acceptable additive selected from among microcrystalline cellulose, mannitol, low-substituted hydroxypropylcellulose (L-HPC), croscarmellose sodium, colloidal silicon dioxide (fumed silica, Aerosil), talc, magnesium stearate, and any combinations thereof.

In another example, the mixture obtained in step (a-1) may comprise sacubitril-valsartan or pharmaceutically acceptable salts thereof as an active ingredient, and a pharmaceutically acceptable additive selected from among microcrystalline cellulose, low-substituted hydroxypropylcellulose (L-HPC), sodium starch glycolate, colloidal silicon dioxide (fumed silica, Aerosil), talc, magnesium stearate, and any combinations thereof.

In the present invention, step (a-2) of preparing granules may be performed according to any granule preparation method known in the art, for example, by a dry granulation method. Specifically, a mixture of sacubitril-valsartan or pharmaceutically acceptable salts thereof as an active ingredient, and pharmaceutically acceptable additives such as an excipient, a disintegrant, or a lubricant may be granulated using a roller compacting or a slugging method. Here, roller compacting refers to a method of preparing granules by compacting powder at a constant pressure while passing the powder between two rollers. The roller compacting method may be performed using a roller compactor. The roller-compacted mixture may then be further subjected to grinding and sieving processes using a fitz-mill, an oscillator, etc., as needed, to obtain granules of an appropriate size.

In the present invention, the post-mixture portion in step (a-3) may comprise a disintegrant, a lubricant, or a mixture thereof, and, if necessary, may further comprise an excipient (diluent) or a binder.

In an example, in step (a-3), the granules obtained in step (a-2), and a post-mixture portion comprising sodium starch glycolate, colloidal silicon dioxide (fumed silica, Aerosil), and magnesium stearate may be adjusted to appropriate contents and mixed together.

In the present invention, the method may further comprise step (a-4) of compressing the mixture into tablets, after step (a-3), depending on the formulation of the final combination formulation.

In the present invention, step (a-4) may be performed using a tablet press commonly used for pressing granules into tablets, for example, a rotary tablet press.

In the present invention, the tablet obtained after compressing the mixture in step (a-4) may have various shapes. For example, the tablet may have an elliptical, rectangular, oval, triangular, almond, peanut, parallelogram, circular, pentagonal, hexagonal, or trapezoidal shape, preferably a circular, oval or parallelogram shape.

In the present invention, the contents of ingredients mixed together when preparing the first release portion overlap with those described above with respect to the first layer of the combination formulation according to the present invention, and thus detailed description thereof will be omitted below to avoid excessive complexity of the specification.

In the present invention, step (b) of preparing the second release portion may comprise step (b-1) of mixing an SGLT-2 inhibitor or a pharmaceutically acceptable salt thereof with a pharmaceutically acceptable additive.

In the present invention, the pharmaceutically acceptable additive in step (b-1) may be at least one selected from the group consisting of an excipient, a disintegrant, and a lubricant.

In the present invention, the excipient (diluent) may be, for example, selected from the group consisting of microcrystalline cellulose, silicified microcrystalline cellulose, magnesium aluminometasilicate, magnesium aluminate silicate, aluminum silicate, sodium silicate, potassium silicate, magnesium silicate, calcium silicate, lactose, lactose hydrate, lactose anhydride, calcium hydrogen phosphate, hydroxypropyl cellulose, hydroxypropyl methylcellulose, dextrin, mannitol, sorbitol, starch, calcium phosphate monohydrate, calcium carbonate, sugars, and any mixtures thereof, without being limited thereto. However, in the present invention, anhydrous calcium phosphate has poor compatibility with dapagliflozin and sacubitril-valsartan, and thus is not included as the excipient (diluent).

In the present invention, the disintegrant may be, for example, selected from the group consisting of crospovidone, methylcellulose, cross-linked carboxymethylcellulose sodium (cross-linked CMC Na, C.CMC Na, or croscarmellose sodium), carboxymethylcellulose calcium, sodium starch glycolate, hydroxypropylcellulose, low-substituted hydroxypropylcellulose (L-HPC), hydroxypropyl methylcellulose, starch, pregelatinized starch, corn starch, potato starch, alginic acid or its sodium salt, and any combinations thereof, without being limited thereto.

In the present invention, the lubricant may be, for example, selected from the group consisting of calcium stearate, colloidal silicon dioxide (fumed silica, Aerosil), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and any combinations thereof, without being limited thereto.

In an example, when the formulation of the combination formulation to be finally prepared is a bilayer tablet, in step (b-1), an SGLT-2 inhibitor or a pharmaceutically acceptable salt thereof as an active ingredient, and a pharmaceutically acceptable additive selected from among microcrystalline cellulose, lactose hydrate, mannitol, hydroxypropyl cellulose, sodium stearyl fumarate, croscarmellose sodium, and any combinations thereof may be adjusted to appropriate contents and mixed together.

In an example, when the formulation of the combination formulation to be finally prepared is a bilayer tablet, in step (b-1), an SGLT-2 inhibitor or a pharmaceutically acceptable salt thereof as an active ingredient, and a pharmaceutically acceptable additive selected from microcrystalline cellulose, mannitol, hydroxypropyl cellulose, sodium stearyl fumarate, sodium croscarmellose, and any combinations thereof may be adjusted to appropriate contents and mixed together.

In addition, in the present invention, when the formulation of the combination formulation to be finally prepared is a bilayer tablet, the method may comprise, after step (b-1), steps of: (b-2) preparing granules; and (b-3) mixing the granules with a post-mixture portion.

In the present invention, the post-mixing portion in step (b-3) may comprise a disintegrant, a lubricant, or a mixture thereof, and, if necessary, may further comprise an excipient (diluent) or a binder, etc.

In an example, in step (b-3), the granules obtained in step (b-2) and the post-mixture portion containing sodium stearyl fumarate and sodium croscarmellose may be adjusted to appropriate contents and mixed together.

In the present invention, in step (c), in order to prepare a combination formulation in the form of a bilayer tablet, the mixture (mixture of the granules and the post-mixture portion) for the first release portion, obtained in step (a-3), and the mixture (mixture of the granules and the post-mixture portion) for the second release portion, obtained in step (b-3) may be used as a mixture for the upper layer of the bilayer tablet and a mixture for the lower layer, respectively, or, conversely, as a mixture for the lower layer and a mixture for the upper layer, respectively, and may be compressed with a tablet press, thereby preparing a bilayer tablet.

Meanwhile, in the present invention, when the formulation of the combination formulation to be finally prepared is a drug-coated tablet, in step (b-1), an SGLT-2 inhibitor or a pharmaceutically acceptable salt thereof as an active ingredient, and a coating agent may be adjusted to appropriate contents and mixed together, thereby preparing a coating solution.

In the present invention, the coating agent is a hydrophilic polymer and may be, for example, at least one selected from the group consisting of hydroxypropyl methylcellulose (HPMC), polyvinyl alcohol (PVA), macrogol polyvinyl alcohol grafted copolymers, polymers of acrylic acid and its salts, polymethacrylate, poly(butylmethacrylate, 2-dimethylaminoethyl methacrylate, methyl methacrylate) copolymers (e.g., Eudragit^{®} E, Evonik), carboxymethylcellulose (sodium salt and calcium salt), ethylcellulose, methylcellulose, hydroxyethylcellulose, ethyl hydroxyethyl cellulose, hydroxypropylcellulose (HPC), L-HPC (low-substituted HPC), polyvinylpyrrolidone (povidone; PVP), vinylpyrrolidone-vinylacetate copolymers (e.g., Kollidon^{®} VA64, BASF), gelatin, guar gum, partially hydrolyzed starch, alginate, xanthan, and mixtures thereof, without being limited thereto. Preferably, the coating agent may be hydroxypropyl methylcellulose (HPMC), polyvinyl alcohol (PVA), a macrogol polyvinyl alcohol grafted copolymer, or a poly(butylmethacrylate, 2-dimethylaminoethyl methacrylate, methyl methacrylate) copolymer (e.g., Eudragit^{®} E, Evonik).

In addition, in the present invention, the coating solvent that is used to prepare the coating solution may be ethanol, methanol, acetone, acetonitrile, tetrahydrofuran, hexane, methylene chloride, isopropyl alcohol, water, etc. or a mixed solvent thereof. Preferably, ethanol, water, or a mixture thereof may be used as the coating solvent.

In the present invention, to prepare the coating solution, at least one selected from the group consisting of plasticizers, lubricants, colorants, fragrances, surfactants, stabilizers, antioxidants, foaming agents, anti-foaming agents, paraffin, and waxes may be used as an additional additive for additional purposes such as coating efficiency, drug stability, appearance, color, protection, maintenance, binding, performance improvement, and preparation process improvement.

In step (c) of the present invention, in order to prepare a combination formulation in the form of a drug-coated tablet, the core containing sacubitril-valsartan or pharmaceutically acceptable salts thereof, obtained in step (a), preferably the tablet-shaped core obtained in step (a-4), may be coated with the coating solution for the second release portion, obtained in step (b-1), thereby preparing a drug-coated tablet in which the core containing sacubitril-valsartan is coated with the SGLT-2 inhibitor drug. Here, the coating may be performed using a conventional method that is used for drug coating in the art, and for example, a coating method using a pan coating machine may be used, without being limited thereto.

In an example, the method for preparing the combination formulation in the form of a bilayer tablet may comprise steps of: (a-1) mixing sacubitril-valsartan or pharmaceutically acceptable salts thereof with a pharmaceutically acceptable additive; (a-2) preparing granules; (a-3) preparing a mixture for the first release portion by mixing the granules with a post-mixture portion; (b-1) mixing a SGLT-2 inhibitor or a pharmaceutically acceptable salt thereof with a pharmaceutically acceptable additive; (b-2) preparing granules; (b-3) preparing a mixture for the second release portion by mixing the granules with a post-mixture portion; and (c) compressing the mixture for the first release portion and the mixture for the second release portion into a bilayer tablet. However, the order in which steps (a-1), (a-2), (a-3), (b-1), (b-2), and (b-3) are performed is not particularly limited.

In another example, the method for preparing the combination formulation in the form of a drug-coated tablet may comprise steps of: (a-1) mixing sacubitril-valsartan or pharmaceutically acceptable salts thereof with a pharmaceutically acceptable additive; (a-2) preparing granules; (a-3) preparing a mixture for the first release portion by mixing the granules with a post-mixture portion; (a-4) compressing the mixture for the first release portion into a uncoated tablet; (b-1) preparing a coating solution by mixing an SGLT-2 inhibitor or a pharmaceutically acceptable salt thereof with a coating agent; and (c) preparing a drug-coated tablet by coating the surface of the uncoated tablet comprising the first release portion with the coating solution. However, the order in which steps (a-1), (a-2), (a-3), (a-4), and (b-1) are performed is not particularly limited.

The preparation method according to the present invention may further comprise a step of coating the obtained combination formulation, especially the combination formulation in the form of a bilayer tablet, with a pharmaceutical immediate-release film coating agent, which may be commonly used in the art, according to a conventional method. In an example, based on the total weight of the uncoated bilayer tablet, about 3% moisture-proof film coating may be performed with Opadry II coating solution, without being limited thereto.

Hereinafter, the present invention will be described in more detail by way of examples. These examples are intended merely to illustrate the present invention in more detail, and it will be apparent to those skilled in the art that the scope of the present invention according to the subject matter of the present invention is not limited by these examples.

### EXAMPLES

### [Comparative Example 1 and 21 Preparation of Single-Layer Tablets Containing Sacubitril-Valsartan and Dapagliflozin

To prepare single combination tablets by physically mixing sacubitril-valsartan and dapagliflozin, sacubitril-valsartan, microcrystalline cellulose, low-substituted hydroxypropyl cellulose, sodium starch glycolate, colloidal silicon dioxide, talc, and magnesium stearate were mixed together according to the composition shown in Table 1 below, and the mixture was compacted using a roller compactor (TFC-LAB, Freund vector) under the conditions of hydraulic pressure of 5 MPa, roller speed of 1 rpm and screw speed of 5 rpm to form dry granules. The granules are sieved through a sieve having a mesh size of 20 mesh (0.86 mm), and then post-mixed with sodium starch glycolate, colloidal silicon dioxide, and magnesium stearate, thereby preparing the final mixture. During the preparation of the dry granules (Comparative Example 1) or during the post-mixing (Comparative Example 1), dapagliflozin, microcrystalline cellulose, D-mannitol, hydroxypropyl cellulose, croscarmellose sodium, and sodium stearyl fumarate were added, thereby preparing the final mixture. Thereafter, the final mixture was compressed using a tablet press (AUTOTAB-200TR, Ichihashi Seiki) at a pressure of 10 kN, thereby preparing single-layer tablets having a hardness of about 15 kP.

**[Table 1]**

| Processes | Ingredients | Contents (mg/tablet) | |
|---|---|---|---|
| | | Comparative Example 1 | Comparative Example 2 |
| | | Mixed tablet (during simultaneous granulation) | Mixed tablet (during post-mixing) |
| Compacting | Sacubitril-valsartan trisodium trihydrate | 228.34 | 228.34 |
| | Dapagliflozin propanediol | 12.30 | - |
| | Microcrystalline cellulose | 53.66 | 53.66 |
| | Low-substituted hydroxypropyl cellulose | 50.00 | 50.00 |
| | Sodium starch glycolate | 20.00 | 20.00 |
| | Colloidal silicon dioxide | 1.60 | 1.60 |
| | Talc | 4.00 | 4.00 |
| | Magnesium stearate | 6.00 | 6.00 |
| Final mixing | Dapagliflozin propanediol | - | 12.30 |
| | Sodium starch glycolate | 20.00 | 20.00 |
| | Colloidal silicon dioxide | 2.40 | 2.40 |
| | Magnesium stearate | 4.00 | 4.00 |

### [Examples 1 and 21 Preparation of Bilayer Tablets Containing Sacubitril-Valsartan and Dapagliflozin

Sacubitril-valsartan, microcrystalline cellulose, low-substituted hydroxypropyl cellulose, sodium starch glycolate, colloidal silicon dioxide, talc, and magnesium stearate were mixed together according to the composition shown in Table 2 below, and the mixture was compacted using a roller compactor (TFC-LAB, Freund vector) under the conditions of hydraulic pressure of 5 MPa, roller speed of 1 rpm and screw speed of 5 rpm to form dry granules which were then sieved through a sieve having a mesh size of 20 mesh (0.86 mm). The sieved granules were post-mixed with sodium starch glycolate, colloidal silicon dioxide, and magnesium stearate, thereby preparing the final mixture containing sacubitril-valsartan. Meanwhile, dapagliflozin, microcrystalline cellulose, lactose hydrate, D-mannitol, hydroxypropylcellulose, croscarmellose sodium, and sodium stearyl fumarate were mixed together, and the mixture was compacted using a roller compactor (TFC-LAB, Freund vector) under the conditions of hydraulic pressure of 5 MPa, roller speed of 1 rpm and screw speed of 5 rpm to form dry granules which were then sieved through a sieve having a mesh size of 20 mesh (0.86 mm). The sieved granules were post-mixed with sodium stearyl fumarate, thereby preparing the final mixture containing dapagliflozin. The final mixture containing sacubitril-valsartan, which constitutes a lower layer and the final mixture containing dapagliflozin, which constitutes an upper layer, were compressed using a tablet press (AUTOTAB-200TR, Ichihashi Seiki) and an oval punch (17.5 mm width x 9 mm length) at a pressure of 10 kN, thereby preparing bilayer tablets (hardness: about 15 kP).

**[Table 2]**

| Processes | | Ingredients | Contents (mg/tablet) | |
|---|---|---|---|---|
| | | | Example 1 | Example 2 |
| | | | Bilayer tablet (microcrystalline cellulose) | Bilayer tablet (lactose hydrate) |
| Sacubitril-valsartan | Compacting | Sacubitril-valsartan trisodium trihydrate | 228.34 | 228.34 |
| | | Microcrystalline cellulose | 53.66 | 53.66 |
| | | Low-substituted hydroxypropyl cellulose | 50.00 | 50.00 |
| | | Sodium starch glycolate | 20.00 | 20.00 |
| | | Colloidal silicon dioxide | 1.60 | 1.60 |
| | | Talc | 4.00 | 4.00 |
| | | Magnesium stearate | 6.00 | 6.00 |
| | Final mixing | Sodium starch glycolate | 20.00 | 20.00 |
| | | Colloidal silicon dioxide | 2.40 | 2.40 |
| | | Magnesium stearate | 4.00 | 4.00 |
| | Total amount | | 390 | 390 |
| Dapagliflozin | Compacting | Dapagliflozin propanediol | 12.30 | 12.30 |
| | | Microcrystalline cellulose | 99.10 | - |
| | | Lactose hydrate | - | 99.10 |
| | | Anhydrous calcium phosphate | - | - |
| | | D-mannitol | 90.00 | 90.00 |
| | | Hydroxypropylcellulose | 10.00 | 10.00 |
| | | Sodium stearyl fumarate | 12.00 | 12.00 |
| | Final mixing | Croscarmellose sodium | 20.00 | 20.00 |
| | | Sodium stearyl fumarate | 8.00 | 8.00 |
| | Total amount | | 251.4 | 251.4 |

### [Example 3] Preparation of Drug-Coated Tablet Containing Sacubitril-Valsartan and Dapagliflozin

The final mixture containing sacubitril-valsartan was prepared in the same manner as in Example 1, and then compressed using a tablet press, thereby preparing uncoated tablets. To prepare a drug coating solution, a coating solution containing dapagliflozin, povidone and a macrogol-polyvinyl alcohol copolymer in 200 mg of purified water and 200 mg of ethanol per tablet was prepared according to the composition shown in Table 3 below. Thereafter, the surface of the uncoated tablet was coated with the coating solution using a high-speed coating system (XENA-IV, Raon) under the conditions of air feed temperature of 55°C, air discharge temperature of 45°C, pan speed of 6 rpm, and air gauge pressure of 2 to 4 kgf/cm².

**[Table 3]**

| Processes | | Ingredients | Contents (mg/tablet) |
|---|---|---|---|
| | | | Example 3 |
| | | | Drug-coated tablet |
| Sacubitril-valsartan | Compacting | Sacubitril-valsartan trisodium trihydrate | 228.34 |
| | | Microcrystalline cellulose | 53.66 |
| | | Low-substituted hydroxypropyl cellulose | 50.00 |
| | | Sodium starch glycolate | 20.00 |
| | | Colloidal silicon dioxide | 1.60 |
| | | Talc | 4.00 |
| | | Magnesium stearate | 6.00 |
| | Final mixing | Sodium starch glycolate | 20.00 |
| | | Colloidal silicon dioxide | 2.40 |
| | | Magnesium stearate | 4.00 |
| | Total amount | | 390 |
| Dapagliflozin | Drug coating | Dapagliflozin propanediol | 12.30 |
| | | Povidone | 2.80 |
| | | Macrogol-polyvinyl alcohol copolymer | 20.00 |
| | Total amount | | 35.1 |

### [Comparative Example 3] Preparation of Cored tablet Containing Sacubitril-Valsartan and Dapagliflozin

The final mixture containing sacubitril-valsartan for an inner core tablet tablet and the final mixture containing dapagliflozin for an outer core tablet were prepared using the same compositions as in Example 1. The final mixture containing sacubitril-valsartan was compressed using a tablet press, thereby preparing inner core tablet tablets. 1/2 of the filling amount of the final mixture containing dapagliflozin, and the inner core tablets were sequentially filled, and then the remaining final mixture containing dapagliflozin was filled, and pressure was applied thereto, thereby preparing cored tablets. At this time, the compression pressure was adjusted between 7 and 10 kN, and no pre-compression was applied.

### [Comparative Example 4] Preparation of polycap Containing Sacubitril-Valsartan Tablet and Dapagliflozin Tablet

The final mixture containing sacubitril-valsartan and the final mixture containing dapagliflozin were prepared using the same compositions as in Example 1. The final mixture of sacubitril-valsartan and the final mixture of dapagliflozin were separately compressed using a tablet press, thereby preparing two single-layer tablets. These two tablets were filled into one capsule using a capsule filling machine (GKF2500, Bosch) under the conditions of air pressure of 5 to 7 bar and filling speed of 30 to 150 C/min, thereby preparing a capsule. As the capsule base material, an HPMC capsule with a water content of about 3 to 7%, gelatin with a water content of about 13 to 16%, and gelatin-PEG with a water content of about 10 to 14% were used.

### [Comparative Example 5] Change of Excipient Ingredients in Bilayer Tablet Containing Sacubitril-Valsartan and Dapagliflozin

In order to examine changes in the dissolution patterns or stability of combination tablets depending on excipient ingredients, a bilayer tablet was prepared according to the same preparation method as in Example 1, except that microcrystalline cellulose used as an excipient in the preparation of the final mixture containing dapagliflozin was changed to anhydrous calcium phosphate as shown in Table 4 below.

**[Table 4]**

| Processes | | Ingredients | Contents (mg/tablet) |
|---|---|---|---|
| | | | Comparative Example 5 |
| | | | Bilayer tablet (anhydrous calcium phosphate) |
| Sacubitril-valsartan | Compacting | Sacubitril-valsartan trisodium trihydrate | 228.34 |
| | | Microcrystalline cellulose | 53.66 |
| | | Low-substituted hydroxypropyl cellulose | 50.00 |
| | | Sodium starch glycolate | 20.00 |
| | | Colloidal silicon dioxide | 1.60 |
| | | Talc | 4.00 |
| | | Magnesium stearate | 6.00 |
| | Final mixing | Sodium starch glycolate | 20.00 |
| | | Colloidal silicon dioxide | 2.40 |
| | | Magnesium stearate | 4.00 |
| | Total amount | | 390 |
| Dapagliflozin | Compacting | Dapagliflozin propanediol | 12.30 |
| | | Microcrystalline cellulose | - |
| | | Lactose hydrate | - |
| | | Anhydrous calcium phosphate | 99.10 |
| | | D-mannitol | 90.00 |
| | | Hydroxypropylcellulose | 10.00 |
| | | Sodium stearyl fumarate | 12.00 |
| | Final mixing | Croscarmellose sodium | 20.00 |
| | | Sodium stearyl fumarate | 8.00 |
| | Total amount | | 251.4 |

### [Experimental Example 1] Dissolution Evaluation for Each Combination formulation

Both sacubitril-valsartan and dapagliflozin ingredients are drugs that have a fast absorption rate with a time to maximum blood concentration (Tmax) of 2 hours or less after drug administration, and have a high bioavailability of about 80% or more. Dapagliflozin is characterized by having high solubility at all pHs, but sacubitril-valsartan is characterized in that its solubility decreases as the pH decreases. Considering these characteristics, the following experiment was conducted to confirm whether both the ingredients would show immediate release in pH 1.2 and pH 6.8 buffers and thus show dissolution profiles similar to those of reference drugs.

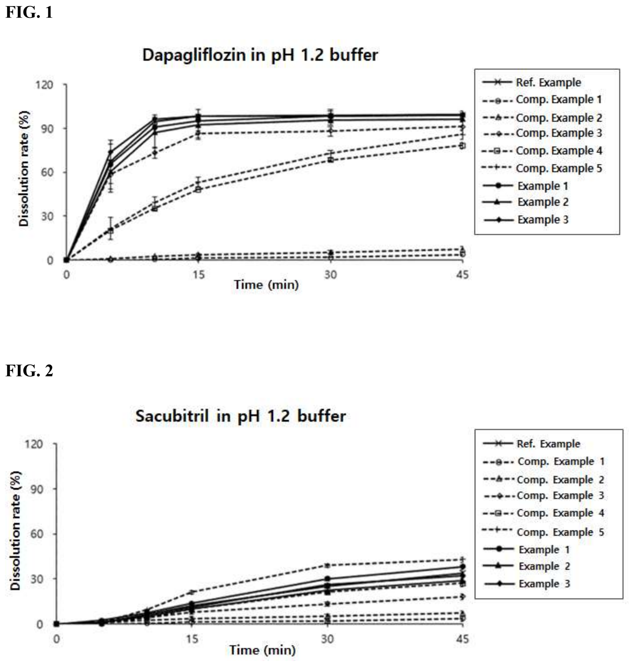

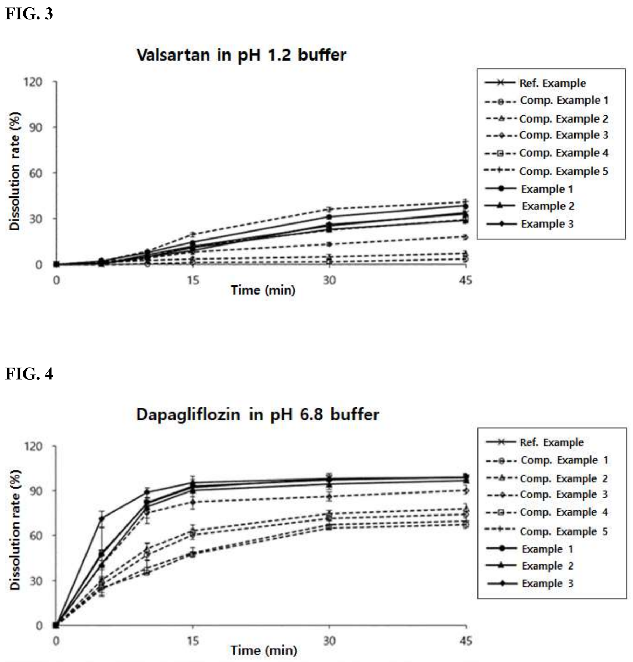

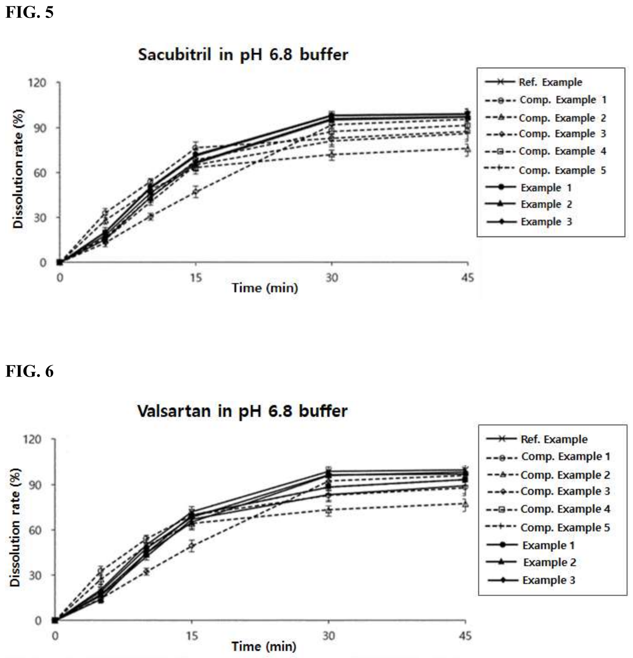

Specifically, for the combination formulations containing 10 mg of dapagliflozin and 200 mg of sacubitril-valsartan, prepared in Examples 1 to 3 and Comparative Examples 1 to 5, dissolution evaluation of dapagliflozin, sacubitril, and valsartan was performed. Considering the absorption location and rate of each drug, the comparison of drug release at low pH and high pH was performed under the following test and analysis conditions. The dissolution rates of each ingredient in the combination formulation in pH 1.2 buffer and pH 6.8 buffer were compared with those of the single reference drugs, Forxiga^{™} Tab (dapagliflozin propanediol hydrate) 10 mg, and Entresto^{™} Tab (sacubitril/valsartan) 200 mg, and the results are shown in Tables 5 to 10 below and FIGS. 1 to 6.

### <Dissolution test conditions>

Dissolution test solutions: pH 1.2 buffer (900 mL), and pH 6.8 buffer (900 mL)
Dissolution device: Paddle
Rotation speed: 50 rpm
Temperature: 37°C

### <Analysis conditions - LC>

Column: Inertsil C8 (4.6 x 150 mm, 5 µm) or column equivalent thereto
Flow rate: 1.5 ml/min
Column temperature: 40°C
Injection volume: 20 µL
Mobile phase: pH2.0 buffer : ACN = 60 : 40
pH 2.0 buffer: Dissolve 3.48 g of potassium dihydrogen phosphate in 1,000 mL of water and adjust the pH to 2.0 with phosphoric acid.

**[Table 5] Time-dependent dissolution rate (%) of dapagliflozin in pH 1.2 buffer**

| Dissolution rate (%) | Comp. Example 1 | Comp. Example 2 | Comp. Example 3 | Comp. Example 4 | Comp. Example 5 | Example 1 | Example 2 | Example 3 | Ref. Example |
|---|---|---|---|---|---|---|---|---|---|
| 0 min | 0.0 | 0.0 | 0.0 | 0.0 | 0.0 | 0.0 | 0.0 | 0.0 | 0.0 |
| 5 min | 0.0 | 1.1 | 58.2 | 20.0 | 21.1 | 65.2 | 60.1 | 73.5 | 66.8 |
| 10 min | 0.5 | 2.5 | 73.2 | 35.2 | 39.2 | 90.5 | 87.2 | 96.2 | 94.6 |
| 15 min | 1.2 | 3.8 | 86.5 | 48.2 | 53.2 | 95.1 | 92.1 | 98.1 | 98.2 |
| 30 min | 1.9 | 5.2 | 88.1 | 68.2 | 72.7 | 98.1 | 95.3 | 99.0 | 98.5 |
| 45 min | 3.7 | 7.2 | 91.2 | 78.1 | 85.7 | 98.9 | 96.1 | 99.2 | 99.1 |
| f₂ (similarity factor) | 4 | 5 | 46 | 20 | 20 | 81 | 64 | 76 | - |

**[Table 6] Time-dependent dissolution rate (%) of sacubitril in pH 1.2 buffer**

| Dissolution rate (%) | Comp. Example 1 | Comp. Example 2 | Comp. Example 3 | Comp. Example 4 | Comp. Example 5 | Example 1 | Example 2 | Example 3 | Ref. Example |
|---|---|---|---|---|---|---|---|---|---|
| 0 min | 0.0 | 0.0 | 0.0 | 0.0 | 0.0 | 0.0 | 0.0 | 0.0 | 0.0 |
| 5 min | 1.1 | 2.1 | 0.5 | 1.5 | 1.0 | 2.3 | 0.8 | 0.7 | 0.6 |
| 10 min | 1.5 | 3.2 | 4.0 | 4.2 | 9.3 | 7.4 | 5.2 | 6.0 | 6.3 |
| 15 min | 2.5 | 4.5 | 8.0 | 10.0 | 21.3 | 13.6 | 10.0 | 11.0 | 12.0 |
| 30 min | 3.7 | 6.7 | 13.3 | 21.3 | 38.8 | 30.1 | 22.3 | 26.0 | 25.3 |
| 45 min | 5.4 | 8.9 | 18.2 | 27.1 | 42.9 | 38.0 | 28.7 | 32.0 | 33.8 |
| f₂ (similarity factor) | 41 | 44 | 54 | 73 | 55 | 76 | 78 | 94 | - |

**[Table 7] Time-dependent dissolution rate (%) of valsartan in pH 1.2 buffer**

| Dissolution rate (%) | Comp. Example 1 | Comp. Example 2 | Comp. Example 3 | Comp. Example 4 | Comp. Example 5 | Example 1 | Example 2 | Example 3 | Ref. Example |
|---|---|---|---|---|---|---|---|---|---|
| 0 min | 0.0 | 0.0 | 0.0 | 0.0 | 0.0 | 0.0 | 0.0 | 0.0 | 0.0 |
| 5 min | 1.0 | 2.2 | 0.1 | 1.8 | 2.4 | 2.5 | 0.7 | 0.3 | 0.6 |
| 10 min | 1.9 | 3.3 | 1.1 | 5.2 | 8.8 | 7.9 | 4.8 | 5.0 | 5.5 |
| 15 min | 2.7 | 4.2 | 1.5 | 11.8 | 19.6 | 14.5 | 11.0 | 9.2 | 10.5 |
| 30 min | 3.8 | 6.6 | 1.3 | 22.5 | 36.1 | 31.1 | 23.0 | 26.1 | 22.6 |
| 45 min | 5.3 | 8.8 | 2.1 | 29.2 | 41.0 | 38.4 | 29.1 | 33.0 | 30.3 |
| f₂ (similarity factor) | 44 | 47 | 41 | 94 | 54 | 64 | 96 | 83 | - |

**[Table 8] Time-dependent dissolution rate (%) of dapagliflozin in pH 6.8 buffer**

| Dissolution rate (%) | Comp. Example 1 | Comp. Example 2 | Comp. Example 3 | Comp. Example 4 | Comp. Example 5 | Example 1 | Example 2 | Example 3 | Ref. Example |
|---|---|---|---|---|---|---|---|---|---|
| 0 min | 0.0 | 0.0 | 0.0 | 0.0 | 0.0 | 0.0 | 0.0 | 0.0 | 0.0 |
| 5 min | 26.8 | 30.2 | 40.0 | 25.1 | 24.2 | 47.1 | 40.5 | 71.2 | 48.0 |
| 10 min | 47.0 | 51.0 | 75.3 | 35.1 | 38.4 | 82.1 | 79.1 | 88.9 | 81.6 |
| 15 min | 60.3 | 63.2 | 82.3 | 47.5 | 48.5 | 93.0 | 90.4 | 95.2 | 92.0 |
| 30 min | 71.5 | 74.8 | 86.1 | 65.2 | 67.5 | 97.1 | 94.2 | 98.0 | 98.2 |
| 45 min | 74.0 | 78.0 | 90.1 | 67.1 | 69.7 | 99.0 | 96.7 | 99.0 | 98.6 |
| f₂ (similarity factor) | 29 | 32 | 54 | 24 | 25 | 95 | 70 | 50 | - |

**[Table 9] Time-dependent dissolution rate (%) of sacubitril in pH 6.8 buffer**

| Dissolution rate (%) | Comp. Example 1 | Comp. Example 2 | Comp. Example 3 | Comp. Example 4 | Comp. Example 5 | Example 1 | Example 2 | Example 3 | Ref. Example |
|---|---|---|---|---|---|---|---|---|---|
| 0 min | 0.0 | 0.0 | 0.0 | 0.0 | 0.0 | 0.0 | 0.0 | 0.0 | 0.0 |
| 5 min | 32.8 | 28.1 | 13.2 | 16.7 | 15.2 | 20.1 | 15.1 | 18.1 | 19.8 |
| 10 min | 53.6 | 48.5 | 30.5 | 43.2 | 40.1 | 50.3 | 43.2 | 46.2 | 49.1 |
| 15 min | 76.4 | 63.2 | 47.2 | 68.2 | 65.0 | 72.1 | 66.0 | 67.0 | 71.0 |
| 30 min | 82.7 | 72.1 | 91.9 | 87.2 | 81.2 | 98.1 | 95.2 | 96.0 | 97.9 |
| 45 min | 87.4 | 75.9 | 95.7 | 91.3 | 86.0 | 99.2 | 97.1 | 97.2 | 98.9 |
| f₂ (similarity factor) | 50 | 41 | 44 | 60 | 50 | 96 | 70 | 79 | - |

**[Table 10] Time-dependent dissolution rate (%) of valsartan in pH 6.8 buffer**

| Dissolution rate (%) | Comp. Example 1 | Comp. Example 2 | Comp. Example 3 | Comp. Example 4 | Comp. Example 5 | Example 1 | Example 2 | Example 3 | Ref. Example |
|---|---|---|---|---|---|---|---|---|---|
| 0 min | 0.0 | 0.0 | 0.0 | 0.0 | 0.0 | 0.0 | 0.0 | 0.0 | 0.0 |
| 5 min | 32.6 | 27.1 | 14.5 | 17.1 | 16.1 | 17.1 | 14.1 | 19.1 | 20.4 |
| 10 min | 54.0 | 49.2 | 32.2 | 44.1 | 42.1 | 44.1 | 44.2 | 47.3 | 49.7 |
| 15 min | 69.8 | 64.2 | 49.5 | 69.6 | 67.1 | 69.6 | 65.0 | 68.2 | 71.7 |
| 30 min | 82.8 | 73.1 | 92.3 | 88.3 | 83.5 | 88.3 | 96.1 | 96.5 | 98.7 |
| 45 min | 87.9 | 77.2 | 96.1 | 93.2 | 89.2 | 93.2 | 98.1 | 96.9 | 99.7 |
| f₂ (similarity factor) | 51 | 42 | 46 | 62 | 53 | 62 | 67 | 83 | - |

As shown in Tables 5 to 10 above, it was confirmed that, in Comparative Examples 1 and 2, which are single-layer tablets containing sacubitril-valsartan and dapagliflozin physically mixed together, sacubitril-valsartan and dapagliflozin were released simultaneously, and in the process in which the two ingredients were dissolved simultaneously in the pH 1.2 buffer and the pH 6.8 buffer, the two components changed into a sticky state and inhibited each other's dissolution, thereby delaying dissolution. In particular, in the pH 1.2 buffer, the dissolution rates of both the ingredients at 45 minutes were found to be 10% or less.

In the case of the cored tablet of Comparative Example 3, designed such that sacubitril-valsartan was distributed in the inner core tablet layer and dapagliflozin was distributed in the outer tablet layer, it was confirmed that disintegration of the inner core tablet layer began after disintegration of the outer tablet layer progressed, and thus dapagliflozin present in the outer tablet layer showed a fast dissolution profile similar to the reference drug, but sacubitril-valsartan present in the inner core tablet layer showed a relatively slow dissolution profile compared to the reference drug.

In the case of the polycap of Comparative Example 4, in which the single tablet of sacubitril-valsartan and the single tablet of dapagliflozin were filled into one capsule, tablet disintegration begins after the capsule was dissolved, and the dissolution rate at the initial stage (5 or 10 minutes) was relatively low compared to that of the reference drug, and the dissolution rate gradually increased after 15 minutes, but the dissolution rate of dapagliflozin was significantly lower than that of the reference drug even after 45 minutes in both pH 1.2 and pH 6.8 buffers.

Meanwhile, in the case of the bilayer tablets of Examples 1 and 2, in which sacubitril-valsartan and dapagliflozin were placed in separate layers, the bilayer tablets showed dissolution profiles similar to those of the reference drugs in pH 1.2 and pH 6.8 buffers with respect to all of the ingredients (sacubitril, valsartan, and dapagliflozin). However, in the case of the bilayer tablet of Comparative Example 5, which contained anhydrous calcium phosphate as an excipient instead of microcrystalline cellulose of Example 1 or lactose hydrate of Example 2, it was shown that the dissolution profile of the dapagliflozin tablet portion was relatively slow compared to that of the reference drug, because anhydrous calcium phosphate is insoluble in water and has a high density.

In the case of the drug-coated tablet of Example 3 in which the core containing sacubitril-valsartan was coated with the drug dapagliflozin, it was confirmed that, since the time when dapagliflozin met the dissolution test solution was the fastest, the initial dissolution rate of dapagliflozin at 5 or 10 minutes tended to be somewhat faster than that of the reference drug, but the dissolution profile of dapagliflozin at a later time point was similar to that of the reference drug, and sacubitril and valsartan showed dissolution profiles similar to those of the reference drug under all conditions (pH 1.2 and pH 6.8 buffers).

### [Experimental Example 21 Evaluation of Stability of Each Combination formulation against Generation of Impurities

Sacubitril-valsartan is relatively stable against heat and water and has good compatibility with commonly used excipients. However, dapagliflozin is more likely to be structurally modified by external factors such that there are more than 80 types of known dapagliflozin impurities. Thus, in the development of dapagliflozin formulations, it is essential to evaluate the stability of dapagliflozin against impurity generation.

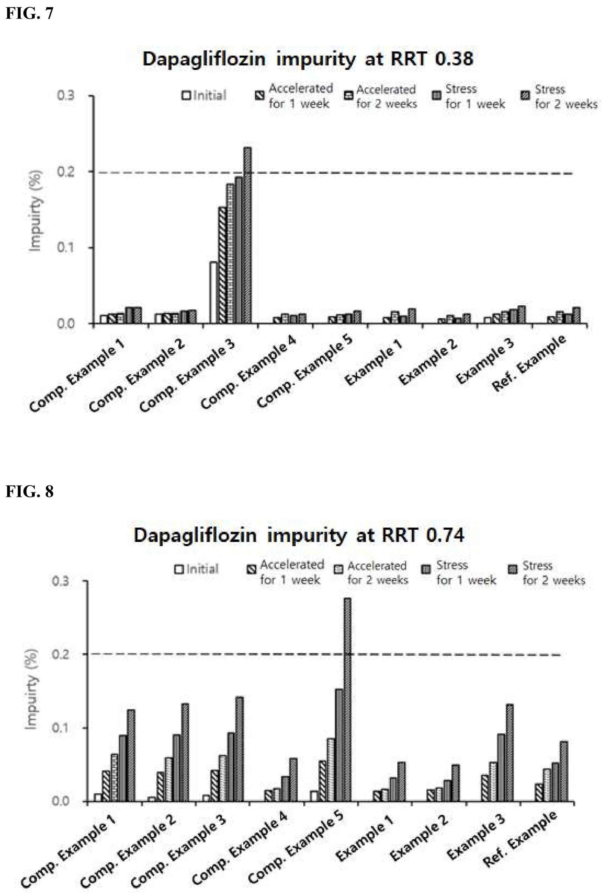

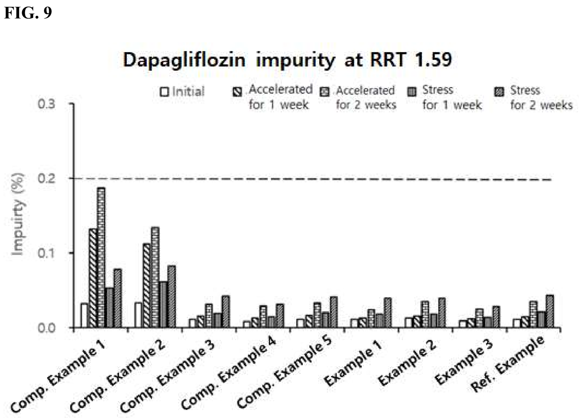

Accordingly, the evaluation of stability against impurity generation was conducted on the combination formulations prepared in Examples 1 to 3 and Comparative Examples 1 to 5. The tendency of increase in dapagliflozin impurities in samples prepared according to the types of formulation and excipient was evaluated by exposure to accelerated conditions (40°C and 75% RH) and stress conditions (60°C) for 2 weeks, and the results are shown in FIGS. 7 to 10. Analysis conditions for impurities are as follows. As a reference example, the results of analysis of impurities for Forxiga Tab^{™} (dapagliflozin propanediol hydrate) 10 mg are shown.

### <Analysis conditions - LC>

Column: Waters X-Bridge C18 (4.6 x 150 mm, 3.5 µm) or column equivalent thereto
Flow rate: 1.0 ml/min
Column temperature: 50°C
Injection volume: 10 µL
Mobile phase: A - ACN : pH 2.5 buffer = 10 : 90, B - MeOH: pH 2.5 buffer = 90 : 10 pH 2.5 buffer: Dissolve 3.12 g of monosodium phosphate dihydrate in 1,000 mL of water and adjust the pH to 2.5 with phosphoric acid.

**[Table 11]**

| Time (min) | A (%) | B (%) |
|---|---|---|
| 0 | 60 | 40 |
| 5 | 60 | 40 |
| 40 | 40 | 60 |
| 50 | 25 | 75 |
| 58 | 25 | 75 |
| 60 | 60 | 40 |
| 70 | 60 | 40 |

As shown in FIGS. 7 to 10, it could be confirmed that, in Comparative Examples 1 and 2, which are combination single tablets in which sacubitril-valsartan and dapagliflozin are not separated from each other but exist while influencing each other, sacubitril-valsartan, which has a high property of absorbing moisture, absorbed moisture during accelerated exposure and affected dapagliflozin, and thus the level of unknown impurities (at RRT 1.59) generated by moisture under accelerated exposure conditions tended to increase.

In the case of the cored tablet of Comparative Example 3, tableting was performed using dapagliflozin as the outer tablet layer surrounding the inner core tablet layer, and during tableting, high compression pressure was transmitted in all directions to maintain the shape. For this reason, unknown impurities (at RRT 0.38), which tended to increase by heat, were detected at high levels from the initial stage, and tended to further increase during exposure to accelerated and stress conditions.

Meanwhile, it could be confirmed that, in the case of the bilayer tablets of Examples 1 and 2, the stability against impurity generation was generally good. However, it could be confirmed that, in the case of the bilayer tablet of Comparative Example 5, which contains anhydrous calcium phosphate as an excipient, the compatibility between dapagliflozin and anhydrous calcium phosphate was not good, and for this reason, unknown impurity (at RRT 0.74) increased significantly when exposed to moisture and heat, and the increase in unknown impurity by heat was larger.

It can be confirmed that, in the case of the drug-coated tablet of Example 3, since dapagliflozin was partially exposed to heat during the drug coating process, the detection level of unknown impurities (at RRT 0.74) tended to be slightly high, but the detection amount was very lower than that in the cored tablet of Comparative Example 3.

### [Example 4] Preparation of Bilayer Tablet Containing Sacubitril-Valsartan and Dapagliflozin

Sacubitril-valsartan, microcrystalline cellulose, D-mannitol, low-substituted hydroxypropyl cellulose, croscarmellose sodium, colloidal silicon dioxide, talc, and magnesium stearate were mixed together according to the composition shown in Table 12 below, and the mixture was fed into a roller compactor, thereby preparing dry granules. The granules were further mixed with croscarmellose sodium and colloidal silicon dioxide, and the resulting mixture was then post-mixed with magnesium stearate, thereby preparing a final mixture containing sacubitril-valsartan (first mixture). Meanwhile, dapagliflozin propanediol, microcrystalline cellulose, D-mannitol, hydroxypropyl cellulose, croscarmellose sodium, and sodium stearyl fumarate were mixed together and fed into a roller compactor, thereby preparing dry granules. Then, the granules were further mixed with croscarmellose sodium, and the resulting mixture was then post-mixed with sodium stearyl fumarate, thereby preparing a final mixture containing dapagliflozin (second mixture). The final mixture containing sacubitril-valsartan, which constitutes a lower layer, and the final mixture containing dapagliflozin, which constitutes an upper layer, were compressed using a tablet press (AUTOTAB-200TR, Ichihashi Seiki) and an oval punch (17.5 mm width x 9 mm length) at a pressure of 10 kN, thereby preparing bilayer tablets (hardness: about 15 kP).

**[Table 12]**

| Processes | | Ingredients | Contents (mg/tablet) |
|---|---|---|---|
| | | | Example 4 |
| | | | Bilayer tablet (microcrystalline cellulose) |
| Sacubitril-valsartan(lower layer portion) | Dry granulation | Sacubitril-valsartan trisodium trihydrate | 228.3 |
| | | Microcrystalline cellulose | 25.0 |
| | | D-mannitol | 19.7 |
| | | Low-substituted hydroxypropyl cellulose | 59.0 |
| | | Croscarmellose sodium | 20.0 |
| | | Colloidal silicon dioxide | 1.5 |
| | | Talc | 4.0 |
| | | Magnesium stearate | 6.0 |
| | Second mixing | Croscarmellose sodium | 20.0 |
| | | Colloidal silicon dioxide | 2.5 |
| | Final mixing | Magnesium stearate | 4.0 |
| | Total amount | | 390 |
| Dapagliflozin (upper layer portion) | Compacting | Dapagliflozin propanediol | 12.3 |
| | | Microcrystalline cellulose | 39.4 |
| | | D-mannitol | 113.6 |
| | | Hydroxypropylcellulose | 10.0 |
| | | Sodium stearyl fumarate | 8.4 |
| | Second mixing | Croscarmellose sodium | 20.0 |
| | Final mixing | Sodium stearyl fumarate | 6.3 |
| | Total amount | | 210 |

### [Experimental Example 3] Dissolution Evaluation of Bilayer Tablet

In order to evaluate the dissolution of each active ingredient in the bilayer tablet containing sacubitril-valsartan and dapagliflozin, prepared in Example 4, the dissolution profile of each active ingredient in pH 1.2 buffer was analyzed in the same manner as Experimental Example 1, and the results are shown in Tables 13 to 15 below.

**[Table 13]**

| Dapagliflozin in pH 1.2 buffer | | Example 4 |
|---|---|---|
| 5 min | Dissolution rate (%) | 44.0 |
| | (min to max) | (38.8 to 50.8) |
| | Deviation (%) | 5.0 |
| 10 min | Dissolution rate (%) | 74.8 |
| | (min to max) | (71.2 to 80.9) |
| | Deviation (%) | 4.2 |
| 15 min | Dissolution rate (%) | 84.8 |
| | (min to max) | (80.2 to 92.6) |
| | Deviation (%) | 5.4 |
| 30 min | Dissolution rate (%) | 96.5 |
| | (min to max) | (94.2 to 98.5) |
| | Deviation (%) | 1.8 |
| 45 min | Dissolution rate (%) | 97.5 |
| | (min to max) | (95.3 to 99.1) |
| | Deviation (%) | 1.7 |

**[Table 14]**

| Sacubitril in pH 1.2 buffer | | Example 4 |
|---|---|---|
| 5 min | Dissolution rate (%) | 3.8 |
| | (min to max) | (2.1 to 4.7) |
| | Deviation (%) | 1.2 |
| 10 min | Dissolution rate (%) | 10.1 |
| | (min to max) | (7.4 to 11.6) |
| | Deviation (%) | 1.9 |
| 15 min | Dissolution rate (%) | 16.4 |
| | (min to max) | (14.0 to 19.1) |
| | Deviation (%) | 2.1 |
| 30 min | Dissolution rate (%) | 27.6 |
| | (min to max) | (26.2 to 29.7) |
| | Deviation (%) | 1.7 |
| 45 min | Dissolution rate (%) | 33.5 |
| | (min to max) | (32.0 to 34.4) |
| | Deviation (%) | 1.1 |

**[Table 15]**

| Valsartan in pH 1.2 buffer | | Example 4 |
|---|---|---|
| 5 min | Dissolution rate (%) | 4.1 |
| | (min to max) | (1.8 to 4.5) |
| | Deviation (%) | 1.1 |
| 10 min | Dissolution rate (%) | 9.8 |
| | (min to max) | (7.3 to 11.4) |
| | Deviation (%) | 1.8 |
| 15 min | Dissolution rate (%) | 15.3 |
| | (min to max) | (13.8 to 18.7) |
| | Deviation (%) | 2.0 |
| 30 min | Dissolution rate (%) | 28.2 |
| | (min to max) | (27.1 to 30.2) |
| | Deviation (%) | 1.8 |
| 45 min | Dissolution rate (%) | 34.1 |
| | (min to max) | (33.1 to 34.5) |
| | Deviation (%) | 1.3 |

As shown in Tables 13 to 15, in the case of the bilayer tablet of Example 4, in which sacubitril-valsartan and dapagliflozin were located in separate layers, the bilayer tablet showed excellent dissolution profiles in pH 1.2 buffer with respect to all the ingredients (sacubitril, valsartan, and dapagliflozin).

### [Examples 5 and 6] Preparation of Bilayer Tablet Containing Sacubitril-Valsartan and Dapagliflozin

Sacubitril-valsartan, microcrystalline cellulose, low-substituted hydroxypropyl cellulose, sodium starch glycolate, colloidal silicon dioxide, talc, and magnesium stearate were mixed together according to the composition shown in Table 16 below, and the mixture was compacted using a roller compactor (TFC-LAB, Freund vector) under the conditions of hydraulic pressure of 5 MPa, roller speed of 1 rpm and screw speed of 5 rpm to form dry granules. The granules are sieved through a sieve having a mesh size of 20 mesh (0.86 mm). The sieved granules were post-mixed with sodium starch glycolate, colloidal silicon dioxide, and magnesium stearate, thereby preparing a final mixture containing sacubitril-valsartan. Meanwhile, dapagliflozin, microcrystalline cellulose, lactose hydrate, D-mannitol, hydroxypropylcellulose, croscarmellose sodium, and sodium stearyl fumarate were mixed together and compacted using a roller compactor (TFC-LAB, Freund vector) under the conditions of hydraulic pressure of 5 MPa, roller speed of 1 rpm and screw speed of 5 rpm to form dry granules which were then sieved through a sieve having a mesh size of 20 mesh (0.86 mm). The sieved granules were post-mixed with sodium stearyl fumarate, thereby preparing a final mixture containing dapagliflozin. The final mixture containing sacubitril-valsartan, which constitutes a lower layer, and the final mixture containing dapagliflozin, which constitutes an upper layer, were compressed using a tablet press (AUTOTAB-200TR, Ichihashi Seiki) and an oval punch (17.5 mm width x 9 mm length) at a pressure of 10 kN, thereby preparing bilayer tablets (hardness: about 15 kP).

**[Table 16]**

| Processes | | Ingredients | Contents (mg/tablet) | |
|---|---|---|---|---|
| | | | Example 5 | Example 6 |
| Sacubitril-valsartan | Compacting | Sacubitril-valsartan trisodium hemi-pentahydrate | 226.2 | 226.2 |
| | | Microcrystalline cellulose | 55.8 | 55.8 |
| | | Low-substituted hydroxypropyl cellulose | 50.00 | 50.00 |
| | | Sodium starch glycolate | 20.00 | 20.00 |
| | | Colloidal silicon dioxide | 1.60 | 1.60 |
| | | Talc | 4.00 | 4.00 |
| | | Magnesium stearate | 6.00 | 6.00 |
| | Final mixing | Sodium starch glycolate | 20.00 | 20.00 |
| | | Colloidal silicon dioxide | 2.40 | 2.40 |
| | | Magnesium stearate | 4.00 | 4.00 |
| | Total amount | | 390 | 390 |
| Dapagliflozin | Compacting | Dapagliflozin propanediol | 12.30 | 12.30 |
| | | Microcrystalline cellulose | 99.10 | - |
| | | Lactose hydrate | - | 99.10 |
| | | Anhydrous calcium phosphate | - | - |
| | | D-mannitol | 90.00 | 90.00 |
| | | Hydroxypropylcellulose | 10.00 | 10.00 |
| | | Sodium stearyl fumarate | 12.00 | 12.00 |
| | Final mixing | Croscarmellose sodium | 20.00 | 20.00 |
| | | Sodium stearyl fumarate | 8.00 | 8.00 |
| | Total amount | | 251.4 | 251.4 |

### [Example 7] Preparation of Drug-Coated Tablet Containing Sacubitril-Valsartan and Dapagliflozin

A final mixture containing sacubitril-valsartan was prepared in the same manner as in Example 5, and then compressed using a tablet press, thereby preparing uncoated tablets. To prepare a drug coating solution, a coating solution containing dapagliflozin, povidone and a macrogol-polyvinyl alcohol copolymer in 200 mg of purified water and 200 mg of ethanol per tablet was prepared according to the composition shown in Table 17 below. Thereafter, the surface of the uncoated tablet was coated with the coating solution using a high-speed coating system (XENA-IV, Raon) under the conditions of air feed temperature of 55°C, air discharge temperature of 45°C, pan speed of 6 rpm, and air gauge pressure of 2 to 4 kgf/cm².

**[Table 17]**

| Processes | | Ingredients | Contents (mg/tablet) |
|---|---|---|---|
| | | | Example 7 |
| Sacubitril-valsartan | Compacting | Sacubitril-valsartan trisodium hemi-pentahydrate | 226.2 |
| | | Microcrystalline cellulose | 55.8 |
| | | Low-substituted hydroxypropyl cellulose | 50.00 |
| | | Sodium starch glycolate | 20.00 |
| | | Colloidal silicon dioxide | 1.60 |
| | | Talc | 4.00 |
| | | Magnesium stearate | 6.00 |
| | Final mixing | Sodium starch glycolate | 20.00 |
| | | Colloidal silicon dioxide | 2.40 |
| | | Magnesium stearate | 4.00 |
| | Total amount | | 390 |
| Dapagliflozin | Drug coating | Dapagliflozin propanediol | 12.30 |
| | | Povidone | 2.80 |
| | | Macrogol-polyvinyl alcohol copolymer | 20.00 |
| | Total amount | | 35.1 |

### [Example 8] Preparation of Bilayer Tablet Containing Sacubitril-Valsartan and Dapagliflozin

Sacubitril-valsartan, microcrystalline cellulose, D-mannitol, low-substituted hydroxypropyl cellulose, croscarmellose sodium, colloidal silicon dioxide, talc, and magnesium stearate were mixed together according to the composition shown in Table 18 below, and the mixture was fed into a roller compactor, thereby preparing dry granules. The granules were further mixed with croscarmellose sodium and colloidal silicon dioxide, and the resulting mixture was then post-mixed with magnesium stearate, thereby preparing a final mixture containing sacubitril-valsartan (first mixture). Meanwhile, dapagliflozin propanediol, microcrystalline cellulose, D-mannitol, hydroxypropyl cellulose, croscarmellose sodium, and sodium stearyl fumarate were mixed together and fed into a roller compactor, thereby preparing dry granules. Then, the granules were further mixed with croscarmellose sodium, and the resulting mixture was then post-mixed with sodium stearyl fumarate, thereby preparing a final mixture containing dapagliflozin (second mixture). The final mixture containing sacubitril-valsartan, which constitutes a lower layer, and the final mixture containing dapagliflozin, which constitutes an upper layer, were compressed using a tablet press (AUTOTAB-200TR, Ichihashi Seiki) and an oval punch (17.5 mm width x 9 mm length) at a pressure of 10 kN, thereby preparing bilayer tablets (hardness: about 15 kP).

**[Table 18]**

| Processes | | Ingredients | Contents (mg/tablet) |
|---|---|---|---|
| | | | Example 8 |
| | Dry granulation | Sacubitril-valsartan trisodium hemi-pentahydrate | 226.2 |
| Sacubitril-valsartan(lower layer portion) | | Microcrystalline cellulose | 27.1 |
| | | D-mannitol | 19.7 |
| | | Low-substituted hydroxypropyl cellulose | 59.0 |
| | | Croscarmellose sodium | 20.0 |
| | | Colloidal silicon dioxide | 1.5 |
| | | Talc | 4.0 |
| | | Magnesium stearate | 6.0 |
| | Second mixing | Croscarmellose sodium | 20.0 |
| | | Colloidal silicon dioxide | 2.5 |
| | Final mixing | Magnesium stearate | 4.0 |
| | Total amount | | 390 |
| Dapagliflozin (upper layer portion) | Compacting | Dapagliflozin propanediol | 12.3 |
| | | Microcrystalline cellulose | 39.4 |
| | | D-mannitol | 113.6 |
| | | Hydroxypropylcellulose | 10.0 |
| | | Sodium stearyl fumarate | 8.4 |
| | Second mixing | Croscarmellose sodium | 20.0 |
| | Final mixing | Sodium stearyl fumarate | 6.3 |
| | Total amount | | 210 |

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### INDUSTRIAL APPLICABILITY

The present invention relates to a pharmaceutical combination formulation containing sacubitril-valsartan and an SGLT-2 inhibitor, which may be used for the treatment of heart failure and ischemic heart disease. The pharmaceutical combination formulation provided by the present invention has excellent stability and dissolution rate.

## Claims

1. A pharmaceutical combination formulation comprising:
(1) a first release portion comprising sacubitril-valsartan or pharmaceutically acceptable salts thereof as an active ingredient; and
(2) a second release portion comprising a sodium-glucose cotransporter-2 (SGLT-2) inhibitor or a pharmaceutically acceptable salt thereof as an active ingredient.

2. The pharmaceutical combination formulation of claim 1, wherein the first release portion and the second release portion are present in a state in which they are physically separated from each other.

3. The pharmaceutical combination formulation of claim 1, wherein the pharmaceutical combination formulation is a multilayer tablet or a drug-coated tablet.

4. The pharmaceutical combination formulation of claim 1, wherein the SGLT-2 inhibitor is selected from the group consisting of dapagliflozin, empagliflozin, canagliflozin, ertugliflozin, sotagliflozin, ipragliflozin, tofogliflozin, luseogliflozin, bexagliflozin, and remogliflozin.

5. The pharmaceutical combination formulation of claim 1, wherein the first or second release portion comprises at least one pharmaceutically acceptable additive selected from the group consisting of an excipient, a disintegrant, and a lubricant.

6. The pharmaceutical combination formulation of claim 5, wherein the excipient is selected from the group consisting of microcrystalline cellulose, silicified microcrystalline cellulose, magnesium aluminometasilicate, magnesium aluminosilicate, aluminum silicate, sodium silicate, potassium silicate, magnesium silicate, calcium silicate, lactose, lactose hydrate, anhydrous lactose, calcium hydrogen phosphate, hydroxypropyl cellulose, hydroxypropyl methylcellulose, dextrin, mannitol, sorbitol, starch, calcium phosphate hydrate, calcium carbonate, sugars, and mixtures thereof.

7. The pharmaceutical combination formulation of claim 5, wherein the disintegrant is selected from the group consisting of crospovidone, methylcellulose, cross-linked carboxymethylcellulose sodium (cross-linked CMC Na, C.CMC Na, or croscarmellose sodium), carboxymethylcellulose calcium, sodium starch glycolate, hydroxypropylcellulose, low-substituted hydroxypropylcellulose (L-HPC), hydroxypropyl methylcellulose, starch, pregelatinized starch, corn starch, potato starch, alginic acid or its sodium salt, and combinations thereof.

8. The pharmaceutical combination formulation of claim 5, wherein the lubricant is selected from the group consisting of calcium stearate, colloidal silicon dioxide (fumed silica), glyceryl monostearate, glyceryl palmitostearate, magnesium stearate, sodium lauryl sulfate, sodium stearyl fumarate, zinc stearate, stearic acid, hydrogenated vegetable oil, polyethylene glycol, sodium benzoate, talc, and combinations thereof.

9. The pharmaceutical combination formulation of claim 1, wherein the second release portion comprises a coating agent.

10. The pharmaceutical combination formulation of claim 9, wherein the coating agent is at least one selected from the group consisting of hydroxypropyl methylcellulose (HPMC), polyvinyl alcohol (PVA), macrogol polyvinyl alcohol grafted copolymers, polymers of acrylic acid and its salts, polymethacrylate, poly(butylmethacrylate, 2-dimethylaminoethyl methacrylate, methyl methacrylate) copolymers, carboxymethylcellulose, ethylcellulose, methylcellulose, hydroxyethylcellulose, ethyl hydroxyethyl cellulose, hydroxypropylcellulose (HPC), L-HPC (low-substituted HPC), polyvinylpyrrolidone (PVP), vinylpyrrolidone-vinylacetate copolymers, gelatin, guar gum, partially hydrolyzed starch, alginate, xanthan, and mixtures thereof.

11. The pharmaceutical combination formulation of claim 1, wherein the first release portion comprises, based on the total weight of the first release portion, 10 to 80 wt% of the active ingredient, 1 to 50 wt% of an excipient, 5 to 50 wt% of a disintegrant, and 0.1 to 20 wt% of a lubricant.

12. The pharmaceutical combination formulation of claim 1, wherein the second release portion comprises, based on the total weight of the second release portion, 0.5 to 50 wt% of the active ingredient, 20 to 95 wt% of an excipient, 0.1 to 20 wt% of a disintegrant, and 0.1 to 20 wt% of a lubricant.

13. The pharmaceutical combination formulation of claim 1, wherein the second release portion comprises, based on the total weight of the second release portion, 10 to 70 wt% of the active ingredient and 30 to 90 wt% of a coating agent.

14. The pharmaceutical combination formulation of claim 1, wherein the first release portion comprises, based on the total weight of the first release portion, 10 to 80 wt% of the active ingredient, 5 to 50 wt% of at least one of microcrystalline cellulose and mannitol, 5 to 30 wt% of low-substituted hydroxypropyl cellulose, 5 to 30 wt% of at least one of sodium starch glycolate and croscarmellose sodium, 0.1 to 10 wt% of colloidal silicon dioxide, 0.1 to 10 wt% of talc, and 0.1 to 10 wt% of magnesium stearate.

15. The pharmaceutical combination formulation of claim 1, wherein the second release portion comprises, based on the total weight of the second release portion, 0.5 to 50 wt% of the active ingredient, 10 to 50 wt% of at least one of microcrystalline cellulose and lactose hydrate, 10 to 50 wt% of mannitol, 0.1 to 20 wt% of hydroxypropyl cellulose, 0.5 to 20 wt% of croscarmellose sodium, and 0.5 to 20 wt% of sodium stearyl fumarate.

16. The pharmaceutical combination formulation of claim 1, wherein the pharmaceutical combination formulation is a bilayer tablet comprising:
a first layer comprising the first release portion comprising the sacubitril-valsartan or pharmaceutically acceptable salts thereof as an active ingredient; and
a second layer comprising the second release portion comprising the SGLT-2 inhibitor or pharmaceutically acceptable salt thereof as an active ingredient.

17. The pharmaceutical combination formulation of claim 1, wherein the pharmaceutical combination formulation is a drug-coated tablet comprising:
a core comprising the first release portion comprising the sacubitril-valsartan or pharmaceutically acceptable salts thereof as an active ingredient; and
a coating layer located on at least a portion of a surface of the core and comprising the second release portion comprising the SGLT-2 inhibitor or pharmaceutically acceptable salt thereof as an active ingredient.

18. The pharmaceutical combination formulation of claim 1, wherein the first release portion comprises granules comprising the sacubitril-valsartan or pharmaceutically acceptable salts thereof as an active ingredient, an excipient, a disintegrant, and a lubricant, and a post-mixture portion comprising a disintegrant and a lubricant, and
the second release portion comprises granules comprising the SGLT-2 inhibitor or pharmaceutically acceptable salt thereof as an active ingredient, an excipient, a disintegrant, and a lubricant, and a post-mixture portion comprising a disintegrant and a lubricant.

19. A method for preparing a pharmaceutical combination formulation, the method comprising steps of:
(a) preparing a first release portion comprising sacubitril-valsartan or pharmaceutically acceptable salts thereof as an active ingredient;
(b) preparing a second release portion comprising an SGLT-2 inhibitor or a pharmaceutically acceptable salt thereof as an active ingredient; and
(c) formulating the first release portion and the second release portion into a solid formulation.

20. The method of claim 19, wherein step (a) of preparing the first release portion comprises steps of: (a-1) mixing sacubitril-valsartan or pharmaceutically acceptable salts thereof and a pharmaceutically acceptable additive; (a-2) preparing granules; and (a-3) mixing the granules and a post-mixture portion.

21. The method of claim 20, further comprising, after step (a-3), step (a-4) of compressing the mixture into tablets.

22. The method of claim 20, wherein step (b) of preparing the second release portion comprises steps of: (b-1) of mixing an SGLT-2 inhibitor or a pharmaceutically acceptable salt thereof with a pharmaceutically acceptable additive; (b-2) preparing granules; and (b-3) mixing the granules and a post-mixture portion.

23. The method of claim 22, wherein step (c) of formulating the first release portion and the second release portion into the single solid formulation comprises a step of compressing the mixture for the first release portion, obtained in step (a-3), and the mixture for the second release portion, obtained in step (b-3), thereby preparing a bilayer tablet.

24. The method of claim 21, wherein step (b) of preparing the second release portion comprises step (b-1) of preparing a coating solution by mixing the SGLT-2 inhibitor or pharmaceutically acceptable salt thereof and a coating agent, and
step (c) of formulating the first release portion and the second release portion into the solid formulation comprises a step of coating a tablet-shaped core, obtained in step (a-4), with the coating solution for the second release portion, obtained in step (b-1).
